(19) Europäisches Patentamt / European Patent Office / Office européen des brevets

(11) **EP 3 820 354 B1**

(12) **FASCICULE DE BREVET EUROPEEN**

(45) Date de publication et mention de la délivrance du brevet:
**18.12.2024 Bulletin 2024/51**

(21) Numéro de dépôt: **19740346.2**

(22) Date de dépôt: **11.07.2019**

(51) Classification Internationale des Brevets (IPC):
*A61B 5/00* (2006.01)    *A61B 8/00* (2006.01)
*G01S 7/52* (2006.01)    *G01S 15/89* (2006.01)

(52) Classification Coopérative des Brevets (CPC):
**A61B 5/7257; A61B 5/0097**

(86) Numéro de dépôt international:
**PCT/EP2019/068740**

(87) Numéro de publication internationale:
**WO 2020/011944 (16.01.2020 Gazette 2020/03)**

(54) **PROCEDÉ ET SYSTÈME D'IMAGERIE ACOUSTO-OPTIQUE PAR RECONSTRUCTION DE FOURIER EN UTILISANT UNE ONDE PLANE COMME ONDE PORTEUSE DE L'ONDE ULTRASONORE**

VERFAHREN ZUR AKUSTOOPTISCHEN ABBILDUNG DURCH FOURIER-REKONSTRUKTION UNTER VERWENDUNG EINER FLACHEN WELLE ALS TRÄGERWELLE DER ULTRASCHALLWELLE

METHOD FOR ACOUSTO-OPTIC IMAGING BY FOURIER RECONSTRUCTION USING A FLAT WAVE AS A CARRIER WAVE OF THE ULTRASONIC WAVE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.07.2018 FR 1856378**

(43) Date de publication de la demande:
**19.05.2021 Bulletin 2021/20**

(73) Titulaires:
• **Centre National de la Recherche Scientifique (CNRS)**
**75016 Paris (FR)**
• **Univ Paris XIII Paris-Nord Villetaneuse**
**93430 Villetaneuse (FR)**
• **INSTITUT NATIONAL DE LA SANTE ET DE LA RECHERCHE MEDICALE - INSERM**
**75013 Paris (FR)**

• **Ecole Supérieure de Physique et de Chimie Industrielles de la Ville de Paris**
**75005 Paris (FR)**

(72) Inventeurs:
• **TUALLE, Jean-Michel**
**92160 ANTONY (FR)**
• **RAMAZ, François**
**77120 COULOMMIERS (FR)**
• **GENNISSON, Jean-Luc**
**95000 CERGY (FR)**
• **BOCOUM, Maimouna**
**94100 SAINT-MAUR-DES-FOSSES (FR)**

(74) Mandataire: **Regimbeau**
**20, rue de Chazelles**
**75847 Paris Cedex 17 (FR)**

(56) Documents cités:
**WO-A1-98/50781    US-A1- 2004 099 815**

**Description**

**Domaine de l'invention**

L'invention concerne un système d'imagerie acousto-optique comprenant un ensemble de transducteurs aptes à produire des ondes ultrasonores modulées dans une zone d'observation.

En particulier, l'invention vise à être utilisée pour imager des zones d'observation in-vivo.

L'invention concerne également un procédé d'imagerie acousto-optique utilisant un tel système d'imagerie.

**Présentation de l'Art Antérieur**

Le principe de l'imagerie acousto-optique (Acousto-Optic Imaging, AOI) implique un faisceau ultrasonore qui induit une modulation de phase sur la lumière diffuse qui le traverse, menant à la génération de photons marqués. Une partie des photons incidents de l'onde lumineuse qui traverse le faisceau ultrasonore deviennent des photons marqués. Les photons marqués sont décalés en fréquence par rapport aux photons incidents avec un écart correspondant à la fréquence ultrasonore du faisceau ultrasonore. WO 2016/193554 A1 concerne les méthodes et systèmes d'imagerie acousto-optique.

Les photons marqués peuvent être détectes par détection incohérente, par exemple en utilisant un filtrage spectral qui les sépare des photons incidents non marqués puis un dispositif de mesure d'intensité lumineuse. Dans ce cas, l'image acousto-optique est construite à partir d'un signal directement proportionnel à la puissance lumineuse des photons marqués détectés.

Il est possible aussi de les détecter par détection interférométrique, par exemple une détection par interférométrie de speckle (tavelures optiques) qui enregistre un signal de battement à la fréquence ultrasonore résultant de l'interférence entre les photons marqués et un faisceau de référence. On connait de l'art antérieur l'exemple d'un ASIC dédié à l'analyse en temps réel des tavelures optique comme dans la demande FR1052535 publiée sous le numéro FR2958430 A1.

Les faisceaux ultrasonores couramment utilisés dans les techniques d'imagerie acousto-optique ont une enveloppe focalisée en un point du milieu à imager. La résolution spatiale dépend de l'extension spatiale de l'onde acoustique, la meilleure résolution spatiale étant obtenue autour de la zone de focalisation, là où l'extension transverse de l'onde acoustique est la plus petite.

La présence d'un point focal de l'onde ultrasonore soulève plusieurs problématiques. L'intensité acoustique, qui est le rapport de la puissance acoustique sur la surface transverse du faisceau, c'est-à-dire la surface transverse à la direction de propagation, est maximale en ce point.

Cette intensité peut être limitée dans le milieu, par exemple pour respecter des normes médicales. La présence du point focal impose alors une limite sur la puissance de l'onde acoustique, ainsi que sur le rapport signal-à-bruit du signal acousto-optique détecté. Par ailleurs, il est difficile de focaliser une onde acoustique à proximité des transducteurs qui la génèrent, et donc de faire une imagerie acousto-optique de qualité à proximité des transducteurs.

**Présentation de l'invention**

Un but général de l'invention est de pallier les inconvénients des systèmes d'imagerie acousto-optique de l'état de la technique.

Notamment, un but de l'invention est de pouvoir augmenter la puissance de l'onde acoustique utilisée pour améliorer la qualité de l'image acousto-optique.

Un autre but encore est de proposer un système d'imagerie acousto-optique capable d'obtenir des images à proximité des transducteurs acoustiques.

Un autre but également est de proposer un système d'imagerie acousto-optique permettant de réaliser des images acousto-optiques en trois dimensions.

Les buts sont atteints dans le cadre de la présente invention grâce à un système d'imagerie acousto-optique d'une zone d'observation d'un milieu, comprenant :

- un ensemble de transducteurs ultrasonores alignés selon au moins une direction d'alignement, aptes à produire ensemble, une onde ultrasonore dans la zone d'observation, se propageant selon une direction de propagation (Dz) perpendiculaire à l'au moins une direction d'alignement des transducteurs;
- une source lumineuse produisant une onde lumineuse incidente, rencontrant ladite onde ultrasonore dans la zone d'observation pour produire des photons marqués ;
- un dispositif de commande configuré pour piloter chaque transducteur ultrasonore avec un signal de commande de fréquence ultrasonore;

- un système de détection d'une partie des photons marqués, lesdits photons marqués portant une modulation temporelle transmise par l'onde ultrasonore rencontrant l'onde lumineuse ;
- un moyen de traitement d'un signal d'acquisition acousto-optique reçu du système de détection, apte à démoduler ledit signal d'acquisition acousto-optique ;

dans lequel le dispositif de commande est configuré pour :

- piloter chaque transducteur ultrasonore par le signal de commande, ledit signal de commande étant constitué d'une porteuse de fréquence ultrasonore,

la porteuse étant identique pour tous les transducteurs, de façon à générer une onde plane se propageant dans la direction de propagation (Dz), et étant modulée par un signal de modulation temporelle périodique de même fréquence temporelle donnée et de même forme pour tous les transducteurs ultrasonores,
ce signal de modulation temporelle ayant un déphasage ($\varphi$) dont la valeur est spécifique à chaque transducteur ultrasonore et qui est une fonction de la position des transducteurs ultrasonores,
de sorte que l'onde ultrasonore plane émise par l'ensemble des transducteurs ultrasonores présente une modulation, qui a une périodicité spatiale selon l'au moins une direction d'alignement des transducteurs dont la valeur est déterminée par la fonction entre le déphasage et la position des transducteurs ultrasonores, - générer différentes ondes ultrasonores correspondant à différentes valeurs de la fréquence temporelle donnée du signal de modulation temporelle et, pour chaque fréquence temporelle donnée, générer différentes fonctions reliant le déphasage et la position des transducteurs ultrasonores,
de façon à déterminer avec le moyen de traitement des composantes de Fourier d'une image acousto-optique de la zone d'observation, et ladite image acousto-optique par transformation de Fourier inverse.

Un tel système d'imagerie acousto-optique permet un protocole d'imagerie sans point focal de l'onde ultrasonore dans le milieu.
Un tel système est avantageusement complété par les différentes caractéristiques suivantes prises seules ou en combinaison :

▪ la position du transducteur ultrasonore est définie par une première coordonnée (x) selon une première direction d'alignement (Dx) orthogonale à la direction de propagation (Dz) et une deuxième coordonnée (y) selon une deuxième direction d'alignement (Dy) orthogonale à la direction de propagation (Dz) et à la première direction d'alignement (Dx), le déphasage ($\varphi$) est une fonction affine des première et deuxième coordonnées (x, y) du transducteur ultrasonore de la forme $\varphi = \varphi_0 + a\,x + b\,y$, où $\varphi_0$, a et b sont des paramètres définissant la relation affine, le choix de ces paramètres permettant de déterminer la période spatiale de la modulation de l'onde ultrasonore plane selon une direction orthogonale à la direction de propagation (Dz).
▪ les transducteurs ultrasonores sont alignés et répartis uniquement selon la première direction d'alignement (Dx), le paramètre b étant nul, et l'onde ultrasonore plane se propage dans un plan d'imagerie défini par la direction de propagation (Dz) et la première direction d'alignement (Dx).
▪ les transducteurs ultrasonores forment un réseau dans le plan défini par les première et deuxième directions d'alignement (Dx, Dy) et l'onde ultrasonore plane se propage dans un volume d'imagerie défini par la direction de propagation (Dz) et les première et deuxième directions d'alignement (Dx, Dy).
▪ le signal de modulation temporelle est un signal carré, un signal sinusoïdal, ou un signal constant par morceaux.
▪ la position du transducteur ultrasonore est définie par une première coordonnée (x) selon une première direction (Dx) orthogonale à la direction de propagation et une deuxième coordonnée (y) selon une deuxième direction d'alignement (Dy) orthogonale à la direction de propagation (Dz) et à la première direction d'alignement (Dx),

ce système d'imagerie permet la mesure de composantes de Fourier de l'image acousto-optique de la zone d'observation associées à une première fréquence spatiale ($v_x$) suivant la première direction d'alignement (Dx), à une deuxième fréquence spatiale ($v_y$) suivant la deuxième direction d'alignement (Dy), et une troisième fréquence spatiale ($v_z$) non nulle suivant la direction de propagation (Dz),
le signal de modulation temporelle est un signal périodique en créneau, dont la fréquence temporelle (f) est égale au produit de la troisième fréquence spatiale ($v_z$) par la vitesse (u) de propagation de l'onde ultrasonore plane dans le milieu, alternant les valeurs +1 et 0 à chaque demi-période temporelle (1/(2f)), ou un signal constant par morceaux prenant alternativement au cours d'une période les valeurs +1, 0, -1, 0 durant quatre durées égales, le signal constant par morceaux étant un signal périodique dont la fréquence temporelle (f/2) est égale à la moitié du produit de la troisième fréquence spatiale ($v_z$) par la vitesse (u),
le déphasage ($\varphi$) est une fonction affine des première et deuxième coordonnées (x, y) du transducteur ultrasonore de la forme $\varphi = \varphi_0 - 2\pi \, v_x \, x - 2\pi \, v_y \, y$,

le signal d'acquisition acousto-optique reçu du système de détection est directement proportionnel à la puissance lumineuse des photons marqués détectés par le système de détection,

le moyen de traitement multiplie le signal d'acquisition acousto-optique reçu du système de détection par une fonction de démodulation temporelle, intègre le résultat sur un intervalle de temps prédéterminé qui est un multiple de la période temporelle de la modulation temporelle (1/f) dans le cas d'un signal périodique en créneau et un multiple de la demi-période temporelle de la modulation temporelle (1/f) dans le cas d'un signal constant par morceaux, pour obtenir un signal démodulé,

la composante de Fourier de l'image acousto-optique de la zone d'observation associée à une première, deuxième et troisième fréquences spatiales $(v_x, v_y, v_z)$ est obtenue à partir d'un signal démodulé ou de plusieurs signaux démodulés correspondant à différentes valeurs du paramètre $\varphi_0$.

- la fonction de démodulation temporelle est $\exp(-2\pi \, j \, f \, t)$, le signal démodulé étant alors un nombre complexe, et la composante de Fourier étant simplement proportionnelle au signal démodulé obtenu pour $\varphi_0 = \pi/2$.

- la fonction de démodulation temporelle est $\sin(2\pi \, f \, t)$, la composante de Fourier étant alors proportionnelle à la somme du signal démodulé obtenu pour $\varphi_0 = 0$ et de j (nombre imaginaire) multiplié par le signal démodulé obtenu pour $\varphi_0 = -\pi/2$ .

- la position du transducteur ultrasonore est définie par une première coordonnée (x) selon une première direction d'alignement (Dx) orthogonale à la direction de propagation (Dz) et une deuxième coordonnée (y) selon une deuxième direction d'alignement (Dy) orthogonale à la direction de propagation (Dz) et à la première direction d'alignement (Dx),

ce système d'imagerie permet la mesure de composantes de Fourier de l'image acousto-optique de la zone d'observation associées à une première fréquence spatiale $(v_x)$ associée à la première direction d'alignement (Dx), à une deuxième fréquence spatiale $(v_y)$ associée à la deuxième direction d'alignement (Dy), et une troisième fréquence spatiale $(v_z)$ non nulle associée à la direction de propagation (Dz),

le signal de modulation temporelle est un signal périodique en créneau, dont la fréquence temporelle (f) est égale à la moitié du produit de la fréquence spatiale $v_z$ par la vitesse (u) de propagation de l'onde ultrasonore plane dans le milieu, alternant les valeurs -1 et +1 à chaque demi-période temporelle (1/(2f)),

le déphasage $(\varphi)$ est une fonction affine des première et deuxième coordonnées (x, y) des transducteurs ultrasonores de la forme $\varphi = \varphi_0 - \pi \, v_x \, x - \pi \, v_y \, y$ ,

le signal d'acquisition acousto-optique reçu du système de détection est un signal interférométrique entre des photons marqués détectés par le système de détection et une onde lumineuse de référence de même longueur d'onde que l'onde lumineuse incidente dans la zone d'observation,

le moyen de traitement multiplie le signal d'acquisition acousto-optique reçu du système de détection par une fonction de démodulation temporelle, intègre le résultat sur un intervalle de temps prédéterminé qui est un multiple de la demi-période temporelle de la modulation temporelle (1/(2f)), pour obtenir un signal démodulé,

le signal démodulé est multiplié par lui-même, ou par un signal démodulé enregistré antérieurement dans des conditions identiques, pour obtenir un signal démodulé élevé au carré, qui est une quantité proportionnelle à l'énergie lumineuse des photons marqués détectés par le système de détection pendant l'intervalle de temps prédéterminé,

la composante de Fourier de l'image acousto-optique de la zone d'observation associée à une première, deuxième et troisième fréquences spatiales $(v_x, v_y, v_z)$ est obtenue à partir de plusieurs signaux démodulés élevés au carré et correspondants à différentes valeurs du paramètre $\varphi_0$.

- la fonction de démodulation temporelle est $\sin(2\pi \, f \, t)$ multiplié par $\cos(2\pi \, f_{US} \, t + \psi)$, où $f_{US}$ est la fréquence de la porteuse du signal de commande de fréquence ultrasonore et $\psi$ une phase, ladite composante de Fourier étant alors proportionnelle à la somme du signal démodulé élevé au carré obtenu pour $\varphi_0 = 0$ et de j (nombre imaginaire) multiplié par le signal démodulé élevé au carré obtenu pour $\varphi_0 = -\pi/4$ , auquel on aura retranché la somme du signal démodulé élevé au carré obtenu pour $\varphi_0 = \pi/2$ et de j (nombre imaginaire) multiplié par le signal démodulé élevé au carré obtenu pour $\varphi_0 = +\pi/4$.

- la reconstruction de l'image par transformation de Fourier inverse est effectuée en utilisant une moyenne de composantes de Fourier obtenues pour différentes valeurs de phase $\psi$, toutes choses étant égales par ailleurs

- l'onde lumineuse incidente rencontrant l'onde ultrasonore plane dans la zone d'observation produite par la source lumineuse est monochromatique.

L'invention porte également sur un procédé d'imagerie acousto-optique d'une zone d'observation d'un milieu qui utilise les systèmes tels qu'on les a décrits dans cette section.

En particulier l'invention porte sur un procédé d'imagerie acousto-optique d'une zone d'observation d'un milieu comprenant les étapes suivantes :

- E1 : une étape de génération d'une onde lumineuse incidente dans la zone d'observation,
- E2 : une étape de génération par un ensemble de transducteurs ultrasonores alignés selon au moins une direction d'alignement, d'une onde ultrasonore plane dans la zone d'observation, avec une fréquence ultrasonore (fus), se propageant selon une direction de propagation (Dz) orthogonale à l'au moins une direction d'alignement des transducteurs,

- E3 : une étape de génération de photons marqués et portant une modulation temporelle transmise par l'onde ultrasonore plane rencontrant l'onde lumineuse,
- E4 : une étape de détection d'une partie des photons marqués et de génération d'un signal d'acquisition acousto-optique par un système de détection,
- E5 : une étape de traitement dudit signal d'acquisition acousto-optique fourni par ledit système de détection comprenant une étape de démodulation temporelle dudit signal d'acquisition acousto-optique en utilisant un signal de démodulation temporelle,

dans lequel :

(i) dans l'étape de génération (E2) tous les transducteurs ultrasonores sont pilotés par un signal de commande constitué d'une porteuse de fréquence ultrasonore ($f_{US}$),

la porteuse étant identique pour tous les transducteurs de façon à générer une onde plane se propageant dans la direction de propagation (Dz), et étant modulée par un signal de modulation temporelle périodique de même fréquence temporelle donnée et de même forme pour tous les transducteurs ultrasonores,
ce signal de modulation temporelle ayant un déphasage ($\varphi$) dont la valeur est spécifique à chaque transducteur ultrasonore et qui est une fonction de la position des transducteurs ultrasonores,
de sorte que l'onde ultrasonore plane émise par l'ensemble des transducteurs ultrasonores présente une modulation, qui a une périodicité spatiale selon l'au moins une direction d'alignement des transducteurs dont la valeur de la période spatiale est déterminée par la fonction entre le déphasage ($\varphi$) et la position des transducteurs ultrasonores;

(ii) le procédé comprenant en outre une étape E6 où les étapes précédentes E1 à E5 sont répétées plusieurs fois, pour différentes valeurs de la fréquence temporelle donnée du signal de modulation temporelle et pour différentes fonctions reliant le déphasage ($\varphi$) et la position du transducteur ultrasonore,

de façon à déterminer des composantes de Fourier d'une image acousto-optique de la zone d'observation, et ladite image acousto-optique par transformation de Fourier inverse.

Avantageusement, mais facultativement, le procédé peut en outre comprendre au moins l'une des étapes suivantes :

- la position du transducteur ultrasonore est définie par une première coordonnée (x) selon une première direction d'alignement (Dx) orthogonale à la direction de propagation (Dz) et une deuxième coordonnée (y) selon une deuxième direction d'alignement (Dy) orthogonale à la direction de propagation (Dz) et à la première direction d'alignement (Dx), le déphasage ($\varphi$) est une fonction affine des première et deuxième coordonnées (x, y) du transducteur ultrasonore : $\varphi = \varphi_0 + a\, x + b\, y$ , où $\varphi_0$ , a et b sont des paramètres définissant la relation affine, le choix de ces paramètres permettant de déterminer la période spatiale de la modulation de l'onde ultrasonore plane selon une direction orthogonale à la direction de propagation (Dz).
- les transducteurs ultrasonores sont alignés et uniquement répartis selon la première direction d'alignement (Dx), respectivement la deuxième direction d'alignement (Dy), le paramètre b, respectivement a, étant nul, et l'onde ultrasonore plane se propage dans un plan d'imagerie défini par la direction de propagation (Dz) et la première direction d'alignement (Dx), respectivement la deuxième direction d'alignement (Dy).
- les transducteurs ultrasonores forment un réseau dans le plan défini par les première et deuxième directions d'alignement (Dx, Dy) et l'onde ultrasonore plane se propage dans un volume d'imagerie défini par la direction de propagation (Dz) et les première et deuxième directions d'alignement (Dx, Dy).
- le signal de modulation temporelle est un signal carré, un signal sinusoïdal, ou un signal constant par morceaux.
- le procédé d'imagerie permet la mesure de composantes de Fourier de l'image acousto-optique de la zone d'observation associées à une première fréquence spatiale ($v_x$) suivant la première direction d'alignement (Dx), à une deuxième fréquence spatiale ($v_y$) suivant la deuxième direction d'alignement (Dy), et une troisième fréquence spatiale ($v_z$) non nulle suivant la direction de propagation (Dz),

le signal de modulation temporelle est un signal périodique en créneau, dont la fréquence temporelle (f) est égale au produit de la troisième fréquence spatiale ($v_z$) par la vitesse (u) de propagation de l'onde ultrasonore plane dans le milieu, alternant les valeurs +1 et 0 à chaque demi-période temporelle (1/(2f)), ou un signal constant par morceaux prenant alternativement au cours d'une période les valeurs +1, 0, -1, 0 durant quatre durées égales, le signal constant par morceaux étant un signal périodique dont la fréquence temporelle (f/2) est égale à la moitié du produit de la troisième fréquence spatiale ($v_z$) par la vitesse (u),

le déphasage ($\varphi$) est une fonction affine des première et deuxième coordonnées (x, y) du transducteur ultrasonore ($\varphi = \varphi_0 - 2\pi v_x x - 2\pi v_y y$), le signal d'acquisition acousto-optique issu de l'étape de détection est directement proportionnel à la puissance lumineuse des photons marqués détectés à l'étape de détection,

l'étape de traitement consiste à multiplier le signal d'acquisition acousto-optique issu de l'étape de détection par une fonction de démodulation temporelle, et à intégrer le résultat sur un intervalle de temps prédéterminé qui est un multiple de la période temporelle de la modulation temporelle (1/f) dans le cas d'un signal périodique en créneau et un multiple de la demi-période temporelle de la modulation temporelle (1/f) dans le cas d'un signal constant par morceaux, pour obtenir un signal démodulé,

la composante de Fourier de l'image acousto-optique de la zone d'observation associée à une première, deuxième et troisième fréquences spatiales ($v_x$, $v_y$, $v_z$) est obtenue à partir d'un signal démodulé ou de plusieurs signaux démodulés correspondant à différentes valeurs du paramètre $\varphi_0$.

- la fonction de démodulation temporelle est $\exp(-2\pi j f t)$, le signal démodulé étant alors un nombre complexe, et la **composante** de Fourier étant simplement proportionnelle au signal démodulé obtenu pour $\varphi_0 = \pi/2$.
- la fonction de démodulation temporelle est $\sin(2\pi f t)$, la composante de Fourier étant alors proportionnelle à la somme du signal démodulé obtenu pour $\varphi_0 = 0$ et de j (nombre imaginaire) multiplié par le signal démodulé obtenu pour $\varphi_0 = -\pi/2$ .
- la position du transducteur ultrasonore est définie par une première coordonnée (x) selon une première direction d'alignement (Dx) orthogonale à la direction de propagation (Dz) et une deuxième coordonnée (y) selon une deuxième direction d'alignement (Dy) orthogonale à la direction de propagation (Dz) et à la première direction d'alignement (Dx),

ce procédé d'imagerie permet la mesure de composantes de Fourier de l'image acousto-optique de la zone d'observation associées à une première fréquence spatiale ($v_x$) suivant la première direction d'alignement (Dx), à une deuxième fréquence spatiale ($v_y$) suivant la deuxième direction d'alignement (Dy), et une troisième fréquence spatiale ($v_z$) non nulle suivant la direction de propagation (Dz),

le signal de modulation temporelle est un signal périodique en créneau, dont la fréquence temporelle (f) est égale à la moitié du produit de la fréquence spatiale $v_z$ par la vitesse (u) de propagation de l'onde ultra-sonore dans le milieu, alternant les valeurs -1 et +1 à chaque demi-période temporelle (1/(2f)),

le déphasage ($\varphi$) est une fonction affine des première et deuxième coordonnées (x, y) du transducteur ultrasonore ($\varphi = \varphi_0 - \pi v_x x - \pi v_y y$),

le signal d'acquisition acousto-optique issu de l'étape de détection est un signal interférométrique entre des photons marqués détectés à l'étape de détection et une onde lumineuse de référence de même longueur d'onde que l'onde lumineuse incidente dans la zone d'observation,

l'étape de traitement consiste à multiplier le signal d'acquisition acousto-optique issu de l'étape de détection par une fonction de démodulation temporelle, et à intégrer le résultat sur un intervalle de temps prédéterminé qui est un multiple de la demi-période temporelle de modulation temporelle (1/(2f)), pour obtenir un signal démodulé,

le signal démodulé est multiplié par lui-même, ou par un signal démodulé enregistré antérieurement dans des conditions identiques, pour obtenir un signal démodulé élevé au carré, qui est une quantité proportionnelle à l'énergie lumineuse des photons marqués détectés à l'étape de détection pendant l'intervalle de temps prédéterminé,

la composante de Fourier de l'image acousto-optique de la zone d'observation associée à une première, deuxième et troisième fréquences spatiales ($v_x$, $v_y$, $v_z$) est obtenue par combinaison linéaire des signaux démodulés élevés au carré et correspondants à différentes valeurs du paramètre $\varphi_0$.

- la fonction de démodulation temporelle est $\sin(2\pi f t)$ multiplié par $\cos(2\pi f_{US} t + \psi)$, où $f_{US}$ est la fréquence de la porteuse du signal de commande de fréquence ultrasonore et $\psi$ un déphasage, la composante de Fourier étant alors proportionnelle à la somme du signal démodulé élevé au carré obtenu pour $\varphi_0 = 0$ et de j (nombre imaginaire) multiplié par le signal démodulé élevé au carré obtenu pour $\varphi_0 = -\pi/4$ , auquel on aura retranché la somme du signal démodulé élevé au carré obtenu pour $\varphi_0=+\pi/2$ et de j (nombre imaginaire) multiplié par le signal démodulé élevé au carré obtenu pour $\varphi_0=+\pi/4$.

- la reconstruction de l'image par transformation de Fourier inverse est effectuée en utilisant une moyenne de composantes de Fourier obtenues pour différentes valeurs du déphasage $\psi$, toutes choses étant égales par ailleurs.
- l'onde lumineuse incidente générée dans la zone d'observation au cours de l'étape E1 est monochromatique.

Il est proposé un procédé et un dispositif d'imagerie acousto-optique utilisant une onde plane comme onde porteuse de l'onde ultrasonore. Ceci permet d'éviter les problématiques liées à la présence d'un point focal de l'onde ultrasonore, comme la difficulté à réaliser une imagerie à proximité du transducteur, ou le dépassement de l'intensité ultrasonore au-delà des normes.

L'onde plane considérée ici peut être une onde de pression se dirigeant selon une direction de propagation (Dz) normale à la surface du milieu à imager. Cette onde peut être créée par un ensemble de transducteurs, et sera définie comme plane dans l'espace ou la zone d'observation si son front d'onde est parallèle aux directions d'alignement des transducteurs. Ladite onde, notée par exemple $A(x, y, z, t)$ peut s'écrire, en fonction de variables spatiales $(x, y, z)$ et du temps $(t)$ sous la forme suivante en notation complexe :

$$A(x, y, z, t) = a(x, y, z) \exp\left[2\,\pi\,j\,f_{US}\,\left(t - \frac{z}{u}\right)\right]$$

où $a(x, y, z)$ désigne l'amplitude de l'onde, exp désigne la fonction exponentielle, j désigne le nombre imaginaire tel que $j^2=-1$, fus est la fréquence de l'onde ultrasonore, et u est la vitesse de propagation de l'onde ultrasonore dans le milieu. Cette onde porteuse peut aussi être une fonction périodique non sinusoïdale, telle que par exemple une fonction créneau. L'amplitude $a(x, y, z)$ peut être une fonction lentement variable de l'espace, en particulier dans le cadre d'une imagerie tridimensionnelle. Dans le cadre d'une imagerie bidimensionnelle dans le plan (x, z), la fonction $a(x, y, z)$ peut être localisée autour de y = 0 afin de matérialiser une coupe dans le plan (x, z).

Il est proposé ici de moduler l'onde porteuse, à savoir ladite onde plane, par une fonction périodique, cette fonction de modulation pouvant par exemple être

- un signal carré de fréquence f / 2 alternant entre les valeurs +1 et -1, dans le cas d'une détection de type interférométrique, ou
- un signal carré de fréquence f alternant entre les valeurs +1 et 0, dans le cas d'une détection de type incohérente, ou
- un signal constant par morceaux de fréquence f / 2 prenant alternativement au cours d'une période les valeurs +1, 0, -1, 0 durant quatre durées égales, dans le cas d'une détection de type incohérente.

La fonction de modulation peut porter un déphasage $\varphi$ dépendant linéairement :

- de la position x de la direction d'alignement Dx selon laquelle sont répartis les transducteurs ultrasonores dans le cas d'une l'imagerie bidimensionnelle, ou
- des positions x et y des directions d'alignement Dx et Dy selon lesquelles sont répartis les transducteurs ultrasonores dans le cas d'une l'imagerie tridimensionnelle.

Une telle modulation peut être mise en oeuvre avec une sonde échographique multi-éléments.

Un signal d'acquisition acousto-optique est généré selon des moyens connus de l'art antérieur pour faire ce type de mesure, mais une étape de démodulation supplémentaire peut être introduite.

**Présentation des figures**

D'autres caractéristiques et avantages de l'invention ressortiront encore de la description qui suit, laquelle est purement illustrative et non limitative, et doit être lue en regard des dessins annexés sur lesquels :

- la figure 1 représente de manière schématique un système d'imagerie acousto-optique selon un mode de réalisation de l'invention ;
- la figure 2 représente de manière schématique un système d'imagerie acousto-optique selon un mode de réalisation de l'invention ;
- la figure 3 représente de manière schématique une onde ultrasonore générée par un système d'imagerie acousto-optique selon un mode de réalisation de l'invention;
- la figure 4a représente la simulation du champ de pression généré par un système d'imagerie selon un mode de réalisation de l'invention ;

- la figure 4b représente la simulation d'une fonction de marquage rendant compte de l'effet d'une modulation et d'une démodulation sur la lumière traitée par un système d'imagerie selon un mode de réalisation de l'invention ;
- la figure 5 représente la simulation de l'image bidimensionnelle d'un objet que l'on aurait obtenue par un système d'imagerie acousto-optique selon un mode de réalisation de l'invention ;
- les figures 6a et 6b représentent les images acousto-optiques d'un même objet obtenues d'une part par un système d'imagerie selon l'art antérieur (figure 6a) et d'autre part par un système d'imagerie selon un mode de réalisation de l'invention (figure 6b);

**Description détaillée**

*Description générale du système d'imagerie acousto-optique*

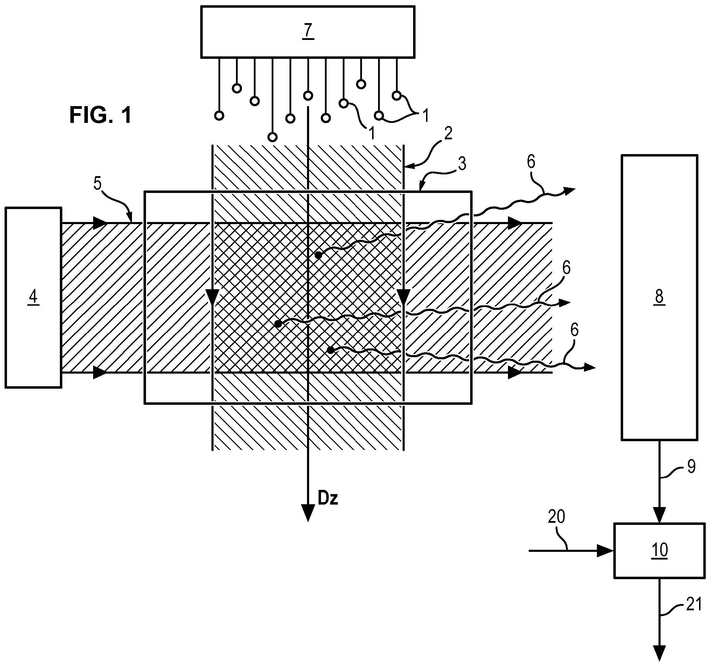

La figure 1 représente un système d'imagerie acousto-optique proposé qui comprend un ensemble de transducteurs ultrasonores 1 et un dispositif de commande 7 configuré pour piloter chaque transducteur ultrasonore 1 avec un signal de commande constitué d'une même porteuse oscillant à une fréquence ultrasonore $f_{US}$.

On désigne dans ce texte par « porteuse » un signal électrique périodique de fréquence ultrasonore fus.

L'ensemble des transducteurs ultrasonores 1 produit une onde ultrasonore 2 qui se propage selon une direction de propagation Dz.

L'onde ultrasonore 2 traverse la zone d'observation 3 du milieu.

Le système d'imagerie acousto-optique comprend une source lumineuse 4 qui produit une onde lumineuse 5.

L'onde lumineuse 5 traverse la zone d'observation 3 du milieu et rencontre l'onde ultrasonore 2.

La rencontre entre l'onde ultrasonore 2 et l'onde lumineuse 5 génère des photons marqués 6. Une partie des photons incidents de l'onde lumineuse 5 qui traverse l'onde ultrasonore 2 deviennent des photons marqués 6. Les photons marqués sont décalés en fréquence par rapport aux photons incidents avec un écart correspondant à la fréquence ultrasonore $f_{US}$.

Le système d'imagerie acousto-optique comprend un système de détection 8 d'une partie des photons marqués 6.

Le système de détection 8 est un système dit de type incohérent et produit un signal d'acquisition acousto-optique 9 directement proportionnel à la puissance lumineuse des photons marqués 6 détectés par le système de détection 8.

Le système de détection 8 peut par exemple filtrer spectralement les photons incidents sur le système de détection et ne laisser passer que les photons marqués. Un tel filtrage spectral peut être réalisé par des dispositifs de type « hole burning » spectral ou bien par des dispositifs d'holographie adaptative faisant intervenir par exemple un cristal photoréfractif.

Les photons marqués qui traversent l'élément de filtrage peuvent être envoyés sur un capteur lumineux qui délivre un signal d'acquisition acousto-optique 9 proportionnel à la puissance lumineuse de ces photons.

Le système d'imagerie acousto-optique comprend un moyen de traitement 10 du signal d'acquisition acousto-optique 9 reçu du système de détection 8.

Le moyen de traitement 10 peut notamment démoduler le signal d'acquisition acousto-optique 9 et à, cet effet, multiplier le signal d'acquisition acousto-optique 9 reçu du système de détection 8 par une fonction de démodulation temporelle 20.

La modulation du signal d'acquisition acousto-optique 9 peut provenir d'une modulation temporelle de l'onde ultrasonore 2 qui a été transmise aux photons marqués 6.

Le moyen de traitement 10 peut produire une image acousto-optique 21 de la zone d'observation du milieu 3.

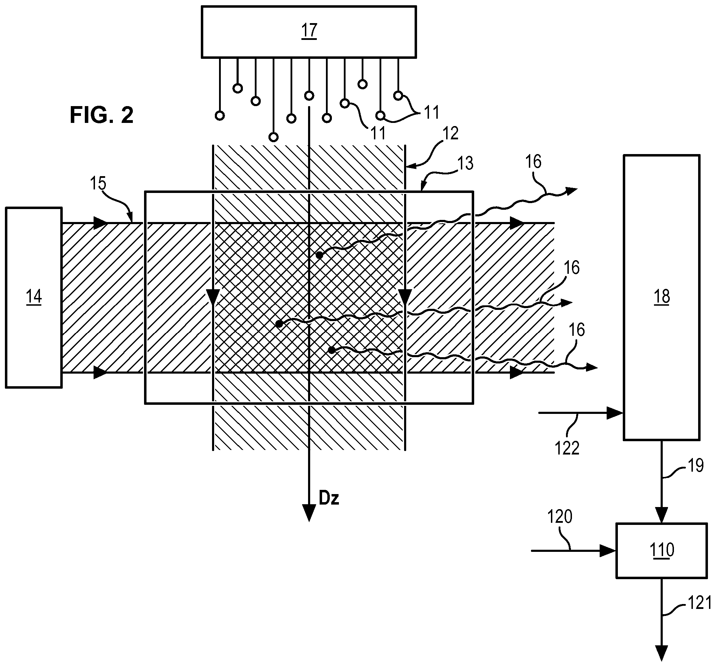

La figure 2 représente un système d'imagerie acousto-optique qui comprend un ensemble de transducteurs ultrasonores 11 piloté par un dispositif de commande 17 et qui produit une onde ultrasonore 12, une source lumineuse 14 qui produit une onde lumineuse 15, ladite onde lumineuse 15 étant de préférence monochromatique pour produire des interférences.

Le système d'imagerie acousto-optique comprend un système de détection 18 d'une partie des photons marqués 16 générés dans la zone d'observation du milieu 13 où se rencontrent l'onde ultrasonore 12 et l'onde lumineuse 15.

Le système de détection 18 est un système dit de type interférométrique, dans lequel les photons marqués 16 détectés par le système de détection 18 sont combinés avec une onde lumineuse de référence 122. Cela génère des interférences lumineuses qui peuvent être mesurées par un capteur lumineux. Le signal obtenu contient la contribution de la lumière diffuse, c'est-à-dire des photons non marqués qui sont rentrés dans le système de détection 18.

L'onde ultrasonore 12 peut être modulée par une modulation temporelle. Cette modulation est alors transmise aux photons marqués 16.

On peut utiliser la modulation temporelle portée par les photons marqués 16 pour éliminer la contribution de la lumière diffuse et obtenir un signal d'acquisition acousto-optique 19.

Il est possible aussi d'obtenir un signal d'acquisition acousto-optique par interférométrie de speckle (tavelures optiques) comme décrit dans la demande FR1052535.

L'onde lumineuse de référence 122 possède la même longueur d'onde que l'onde lumineuse 15.

L'onde lumineuse 122 peut aussi être décalée en fréquence par rapport à l'onde lumineuse 15, le traitement du signal d'acquisition acousto-optique 19 devant alors être adapté en conséquence. Le système d'imagerie acousto-optique comprend un moyen de traitement 110 du signal d'acquisition acousto-optique 19 reçu du système de détection 18.

Le moyen de traitement 110 peut notamment démoduler le signal d'acquisition acousto-optique 19 et à, cet effet multiplier le signal d'acquisition acousto-optique 19 reçu du système de détection 18 par une fonction de démodulation temporelle 120.

Le moyen de traitement 110 peut produire une image acousto-optique 121 de la zone d'observation du milieu 13.

*Modulation de l'onde ultrasonore*

Dans les systèmes d'imagerie acousto-optique correspondant aux figures 1 et 2, le signal d'acquisition acousto-optique 9, 19 est modulé car l'onde ultrasonore 2, 12 porte une modulation qui est transmise aux photons marqués 6, 16.

La modulation de l'onde ultrasonore peut être réalisée lorsque le dispositif de commande 7, 17 est configuré pour piloter chaque transducteur ultrasonore par un signal de commande constitué d'une même porteuse modulée par un signal de modulation temporelle périodique fréquence temporelle donnée.

En particulier, le signal de commande utilisé par le dispositif de commande 7, 17 pour piloter chaque transducteur ultrasonore 1,11 peut être un signal carré oscillant à une fréquence ultrasonore $f_{US}$, modulé par un signal de modulation temporelle de fréquence $f = \dfrac{1}{P_m}$ .

Le signal de modulation temporelle peut être un signal carré ou sinusoïdal. Il peut notamment être :

- un signal carré de fréquence *f* alternant entre les valeurs -1 et +1 , pour le cas des systèmes de détection de type interférométrique ;
- un signal carré de fréquence *f* alternant entre les valeurs 0 et +1 pour le cas des systèmes de détection de type incohérent ; ou
- un signal constant par morceaux de fréquence *f*/2, prenant alternativement au cours d'une période les valeurs +1, 0, -1, 0 durant quatre durées égales, pour le cas des systèmes de détection de type incohérent.

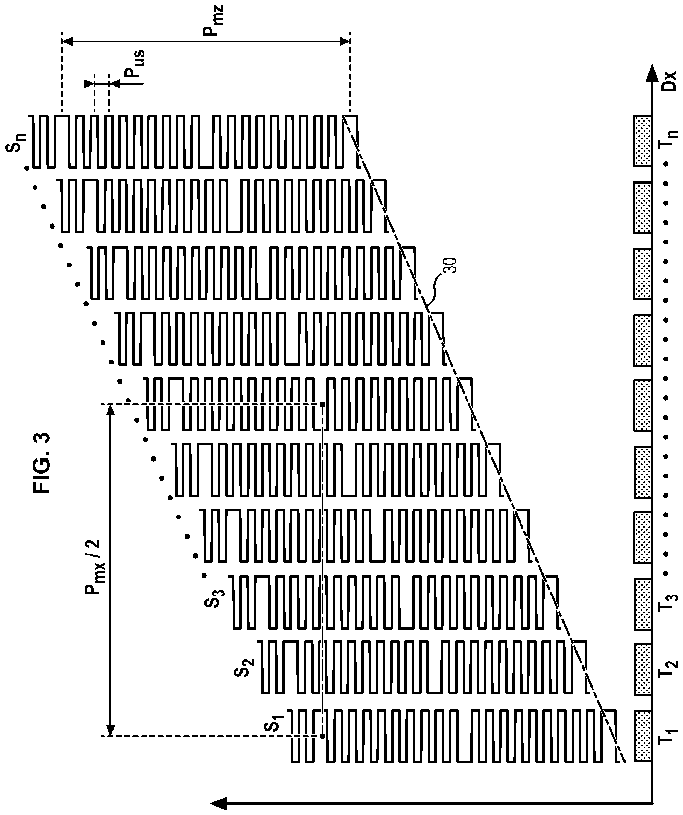

La figure 3 représente de manière schématique une onde ultrasonore qui porte une modulation dans lequel le signal de modulation temporelle est un signal carré de fréquence *f* alternant entre les valeurs -1 et +1.

En référence à la figure 3 un système d'imagerie acousto-optique comprend un ensemble de transducteurs $T_1$, $T_2 ... T_n$ répartis selon une direction d'alignement Dx. Les transducteurs $T_1$, $T_2 ... T_n$ émettent les signaux ultrasonores $S_1$, $S_2 ... S_n$ dans la direction de propagation Dz qui peut être perpendiculaire à la direction d'alignement Dx.

Les signaux $S_1$, $S_2 ... S_n$ ont été représentés en fonction du temps t. Ils possèdent tous la même forme, et dépendent de différentes périodes caractéristiques :

- la période ultrasonore Pus correspond à la fréquence ultrasonore fus, soit $f_{US} = \dfrac{1}{P_{US}}$
- la période de modulation Pmz est la période du signal de modulation temporelle de fréquence *f* des signaux $S_1$, $S_2 ... S_n$ , soit $f = \dfrac{1}{P_{mz}}$ .

Les porteuses des signaux $S_1$, $S_2 ... S_n$ oscillent en phase à la fréquence ultrasonore fus, en revanche leur modulation temporelle est décalée d'un signal à l'autre. La valeur du déphasage de la modulation temporelle est spécifique à chaque transducteur ultrasonore et est une fonction de la position du transducteur ultrasonore.

En particulier, le déphasage dans la modulation temporelle de deux signaux peut dépendre linéairement de la distance selon la direction d'alignement Dx séparant les transducteurs ultrasonores correspondants. La courbe 30 sur la figure 3 représente une dépendance linéaire du déphasage ou retard dans la modulation temporelle en fonction de la position du transducteur selon la direction d'alignement Dx.

La position du transducteur ultrasonore peut être définie par une première coordonnée x selon une première direction d'alignement Dx orthogonale à la direction de propagation Dz. Le déphasage $\varphi$ est une fonction affine de la première coordonnée x du transducteur ultrasonore de la forme $\varphi = \varphi_0 + a\,x$, où $\varphi_0$ et a sont des paramètres définissant la relation affine.

Dans cette situation, il est possible de définir une période spatiale Pmx qui correspond à une distance séparant deux transducteurs qui oscillent en phase à la fréquence ultrasonore fus, avec des modulations temporelles décalées d'une période de modulation Pmz.

Le contrôle individuel des transducteurs $T_1$, $T_2$ ... $T_n$ et la génération de signaux ultrasonores $S_1$, $S_2$ ... $S_n$ dont on contrôle le déphasage permet de générer une onde acoustique qui ne se focalise pas au cours de sa propagation selon la direction Dz.

L'introduction de la modulation temporelle d'une part et l'introduction d'un déphasage dans la modulation temporelle permet d'introduire dans cette onde acoustique des fréquences spatiales :

- Une première fréquence spatiale qui est associée à la direction d'alignement Dx et à la dépendance linéaire du déphasage.
- Une autre fréquence spatiale qui est associée à la direction Dz et à la fréquence de modulation temporelle.

*Modulation de l'onde ultrasonore avec trois fréquences spatiales*

La position du transducteur ultrasonore peut être définie en outre par une deuxième coordonnée y selon une deuxième direction d'alignement Dy orthogonale à la direction de propagation Dz et à la première direction d'alignement Dx.

Le déphasage $\varphi$ peut être une fonction affine des première et deuxième coordonnées x, y du transducteur ultrasonore de la forme $\varphi = \varphi_0 + a\,x + b\,y$, où $\psi_0$, a et b sont des paramètres définissant la relation affine.

Le contrôle individuel des transducteurs et la génération de signaux ultrasonores dont on contrôle le déphasage permet de générer une onde acoustique plane qui ne se focalise pas au cours de sa propagation selon la direction Dz.

Plus précisément, c'est le fait de piloter les transducteurs par la même porteuse qui permet de générer une onde acoustique plane.

L'onde acoustique plane générée est utilisée comme « onde porteuse » d'une modulation.

L'introduction d'un déphasage dans la modulation temporelle de la porteuse, sous cette forme particulière permet d'introduire dans cette onde acoustique les fréquences spatiales :

- Une première fréquence spatiale qui est associée à la direction d'alignement Dx et à la dépendance linéaire du déphasage.
- Une deuxième fréquence spatiale qui est associée à la direction d'alignement Dy et à la dépendance linéaire du déphasage.
- Une troisième fréquence spatiale est associée à la direction de propagation Dz et à la fréquence de modulation temporelle.

Dans le cas où b, respectivement a, est nul dans l'expression $\varphi = \varphi_0 + a\,x + b\,y$, la modulation de l'onde ultrasonore ne comporte que deux fréquences spatiales et l'onde ultrasonore se propage dans un plan d'imagerie défini par la direction de propagation Dz et la première direction d'alignement Dx, respectivement la deuxième direction d'alignement Dy.

Les transducteurs ultrasonores peuvent former un réseau dans le plan défini par les première et deuxième directions d'alignement Dx, Dy. Dans ce cas l'onde ultrasonore se propage dans un volume d'imagerie défini par la direction de propagation Dz et les première et deuxième directions d'alignement Dx, Dy.

*Mesure et démodulation du signal acousto-optique*

L'onde ultrasonore 2,12 somme de l'ensemble des ondes $S_1$, $S_2$,... $S_n$ se propage selon la direction Dz, sans présenter un phénomène de focalisation, jusqu'à rencontrer l'onde lumineuse 5,15 dans la zone d'observation du milieu à imager. Des photons marqués 6,16 sont générés et portent une modulation temporelle transmise par l'onde ultrasonore 2,12 rencontrant l'onde lumineuse 5,15. Une partie des photons marqués générés dans cette zone entrent dans le système de détection.

Le système de détection peut être issu de l'art antérieur, de type incohérent ou interférométrique.

Une démodulation spécifique est nécessaire pour prendre en compte la modulation et les fréquences spatiales portées par les photons marqués 6,16. Une telle démodulation peut faire appel à une fonction de démodulation $h_m(t)$.

On peut notamment utiliser pour un système de détection de type interférométrique :

- une multiplication par le produit d'une fonction sinusoïdale de fréquence $f_{US}$ et par une fonction de démodulation $hm(t)$ de fréquence $f$, par exemple $\cos(2\pi f_{US}t + \psi) * \sin(2\pi ft)$, où $\psi$ est une phase.
- une intégration sur une période $T$ telle que $fT$ est un entier, ou un filtrage spectral passe-bas.

une élévation au carré, c'est-à-dire une multiplication du résultat de l'intégration (ou du filtrage) par lui-même, avantageusement suivie d'une moyenne sur plusieurs acquisitions, de façon à obtenir un signal acousto-optique démodulé élevé au carré $\Phi_d^2$, qui est proportionnel l'énergie des photons marqués. Ce signal constitue le signal d'acquisition acousto-optique 19. Une alternative pourra consister à multiplier le résultat de l'intégration (ou du filtrage) par un signal démodulé enregistré antérieurement dans des conditions identiques.

On peut notamment utiliser pour un système de détection de type incohérent :

- une multiplication par une fonction de démodulation $h_m(t)$ de fréquence $f$, telle que par exemple $h_m(t) = \sin(2\pi ft)$ ou $h_m(t) = \exp(-2\pi jft)$. Dans cette dernière expression, la constante j correspond au nombre imaginaire tel que $(j)^2 = -1$.
- une intégration sur une période $T$ telle que $fT$ est un entier, ou un filtrage spectral passe-bas de façon à obtenir un signal acousto-optique démodulé $\Phi_d$. Ce signal constitue le signal d'acquisition acousto-optique 9.

Quel que soit le type de système de détection utilisé, à l'issue de la démodulation, on obtient un signal d'acquisition acousto-optique $\Phi_d$ référencé 9 sur la figure 1 ou $\Phi_d^2$ référencé 19 sur la figure 2.

Ce signal d'acquisition acousto-optique correspond à l'intégrale sur la zone d'observation du produit de l'image acousto-optique S(x,y,z) que l'on cherche à reconstruire par une fonction de marquage M(x,y,z).

Une fonction de marquage est une fonction qui rend compte de l'effet de la modulation et de la démodulation sur les photons marqués issus d'un point de coordonnées (x,y,z) dans la zone d'observation du milieu qui est imagée.

Selon le type de système de détection utilisé et le type de démodulation appliquée, différentes fonctions de marquage acousto-optiques de la lumière M(x,y,z) peuvent être obtenues.

La fonction de marquage dépend de la modulation de l'onde ultrasonore et plus particulièrement, d'une part, des paramètres $(\varphi_0, a, b)$ de la dépendance linéaire du déphasage $\varphi = \varphi_0 + a\,x + b\,y$, et d'autre part de la fréquence de modulation temporelle $f$.

Le signal d'acquisition acousto-optique dépend des mêmes paramètres $\varphi_0$, a, b, et $f$ que la fonction de marquage M, et peut donc s'écrire sous la forme $\Phi_d(\varphi_0, a, b, f)$ (éventuellement sous la forme $\Phi_d^2(\varphi0, a, b, f)$ pour le cas interférométrique).

A partir de l'expression de la fonction de marquage, on peut établir les signaux d'acquisition acousto-optiques à acquérir, et les opérations algébriques à effectuer sur ces signaux démodulés, pour déterminer une composante de la transformée de Fourier de l'image acousto-optique.

La composante de Fourrier $\tilde{S}(\upsilon_x, \upsilon_y, \upsilon_z)$ de l'image acousto-optique correspond aux fréquences spatiales $\upsilon_x$ associée à la première direction d'alignement Dx, $\upsilon_y$ associée à la deuxième direction d'alignement Dy, et $\upsilon_z$ associée à la direction de propagation Dz.

*Mesure et démodulation du signal acousto-optique avec deux fréquences spatiales*

Ce cas correspond à la situation dans laquelle a ou bien b, est nul dans l'expression $\varphi = \varphi_0 + a\,x + by$.

On choisit de décrire la situation où le paramètre b est nul, l'ensemble des éléments proposés pouvant bien évidemment s'appliquer à n'importe quelle direction d'alignement Dx orthogonale à la direction de propagation Dz.

Dans cette situation la fonction de marquage, qui peut s'écrire M(x,z), est une fonction qui rend compte de l'effet de la modulation et de la démodulation sur les photons marqués issus d'un point de coordonnées (x,z) dans la zone d'observation du milieu qui est imagée.

M(x,z) dépend de la modulation de l'onde ultrasonore et plus particulièrement, d'une part, des paramètres $(\varphi_0, a)$ de la dépendance linéaire du déphasage $\varphi = \varphi_0 + a\,x$, et d'autre part de la fréquence de modulation temporelle $f$.

Le signal d'acquisition acousto-optique correspond à l'intégrale sur la zone d'observation du produit de la fonction de marquage M(x,z) par l'image acousto-optique S(x,z) que l'on cherche à reconstruire.

Le signal d'acquisition acousto-optique dépend des mêmes paramètres $\varphi_0$, a, et $f$ que la fonction de marquage M, et peut donc s'écrire sous la forme $\Phi_d(\varphi_0, a, f)$ (éventuellement sous la forme $\Phi_d^2(\varphi0, a, b, f)$ pour le cas interférométrique).

A partir de l'expression de la fonction de marquage, on peut établir les signaux d'acquisition acousto-optiques à acquérir, et les opérations algébriques à effectuer sur ces signaux démodulés, pour déterminer une composante de la transformée de Fourier de l'image acousto-optique.

La composante de Fourrier $\tilde{S}(\upsilon_x, \upsilon_z)$ de l'image acousto-optique correspond aux fréquences spatiales $\upsilon_x$ associée à la première direction d'alignement Dx et $\upsilon_z$ associée à la direction de propagation Dz.

Pour un système de détection de type interférométrique où :

- la porteuse du signal de commande est un signal carré de fréquence ultrasonore $f_{US}$,

- le signal de modulation temporelle est un signal carré de fréquence $f = \frac{1}{P_m}$ alternant entre les valeurs -1 et +1 à chaque demi-période temporelle $\frac{P_m}{2} = \frac{1}{2f}$

- le déphasage $\varphi$ dépend de la coordonnée x selon $\varphi = \varphi_0 + a\,x$

- la fonction de démodulation est $\cos(2\pi f_{US}t + \psi) * \sin(2\pi ft)$ où $\psi$ est une phase,

on peut utiliser

- une fonction de marquage associée au point de coordonnées (x,z) proportionnelle à l'expression

$$M(x,z) = \cos^2\left(\frac{2\pi f z}{u} - \varphi_0 - ax\right) = \frac{1}{2}\left(\cos\left(2\pi\left(\frac{2f}{u}z - \frac{a}{\pi}x\right) - 2\varphi_0\right) + 1\right)$$

- une composante de Fourier $\tilde{S}(v_x, v_z)$ de l'image acousto-optique qui peut être déterminée en choisissant a = $-\pi v_x$, $f = \frac{u v_z}{2}$ et à partir du calcul suivant :

$$\tilde{S}(v_x, v_z) = \Phi_d^{\,2}(0, a, f) + j.\Phi_d^{\,2}\left(-\frac{\pi}{4}, a, f\right)$$

$$- \left(\Phi_d^{\,2}\left(+\frac{\pi}{2}, a, f\right) + j.\Phi_d^{\,2}\left(+\frac{\pi}{4}, a, f\right)\right)$$

- une moyenne des composantes de Fourier obtenues pour différentes valeurs de phase $\psi$, voire pour plusieurs acquisitions, toutes choses étant égales par ailleurs . Cette moyenne est utile pour éliminer tout effet lié à l'onde porteuse, et peut également améliorer, par effet statistique, le rapport signal à bruit.

L'expression toutes choses étant égales par ailleurs signifient que tous les objets et les paramètres en dehors de la phase $\psi$ sont maintenus constants : signaux de modulation, fréquences, valeurs de $\varphi_0$, a et b.....

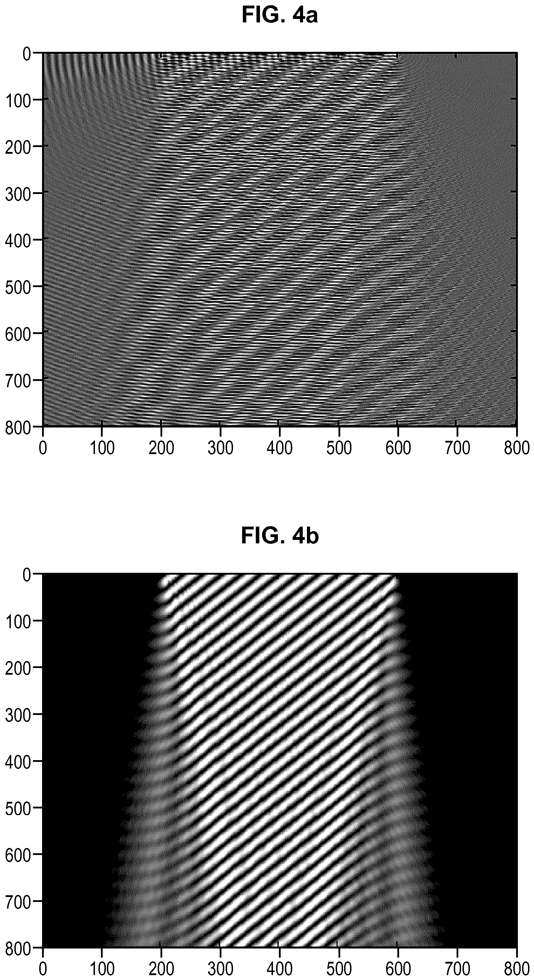

La figure 4a représente la simulation du champ de pression généré par les transducteurs d'un système d'imagerie dans la situation d'un signal acousto-optique avec deux fréquences spatiales.

La figure 4b représente la simulation de la fonction de marquage associée à l'onde sonore simulée sur la figure 4a.

La première direction d'alignement Dx correspond à l'axe supérieur horizontal des figures 4a et 4b.

La direction de propagation Dz correspond à la direction verticale horizontal des figures 4a et 4b.

L'onde sonore générée par les transducteurs présente une extension spatiale correspondant environ au couloir vertical des figures 4a et 4b compris entre les valeurs 200 et 600 référencées sur les axes horizontaux. La section horizontale de ce couloir est sensiblement constante, ce qui correspond au fait que l'onde ultrasonore ne présente pas un phénomène de focalisation.

Les couloirs des figures 4a et 4b sont parcourus de stries obliques. La distance verticale entre deux stries correspond à la périodicité associée à la direction de propagation Dz, et la pente des stries obliques correspond à la périodicité associée à la direction d'alignement Dx.

Pour un système de détection de type incohérent où :

- la porteuse du signal de commande est un signal carré de fréquence ultrasonore $f_{US}$,

- le signal de modulation temporelle est un signal carré de fréquence $f = \frac{1}{P_m}$ alternant entre les valeurs +1 et 0 à chaque demi-période temporelle $\frac{P_m}{2} = \frac{1}{2f}$ , ou un signal constant par morceaux prenant alternativement au cours d'une période les valeurs +1, 0, -1, 0 durant quatre durées égales, le signal constant par morceaux étant un signal périodique de fréquence $\frac{f}{2} = \frac{1}{2P_m}$ .

- le déphasage φ dépend de la coordonnée x selon φ = φ$_0$ + a x
- la fonction de démodulation est sin($2\pi ft$),

on peut utiliser

- une fonction de marquage associé au point de coordonnées (x,z) proportionnelle à l'expression

$$M(x,z) = cos\left(\frac{2\pi fz}{u} - \varphi_0 - ax\right)$$

- une composante de Fourrier $\tilde{S}(v_x, v_z)$ de l'image acousto-optique qui peut être déterminée en choisissant a = -$2\pi v_x$, f = $uv_z$ et à partir du calcul suivant :

$$\tilde{S}(v_x, v_z) = \Phi_d(0, a, f) + j.\Phi_d\left(-\frac{\pi}{2}, a, f\right)$$

Pour un système de détection de type incohérent où :

- la porteuse du signal de commande est un signal carré de fréquence ultrasonore f$_{US}$,

- le signal de modulation temporelle est un signal carré de fréquence $f = \frac{1}{P_m}$ alternant entre les valeurs +1 et 0 à chaque demi-période temporelle $\frac{P_m}{2} = \frac{1}{2f}$, ou un signal constant par morceaux prenant alternativement au cours d'une période les valeurs +1, 0, -1, 0 durant quatre durées égales, le signal constant par morceaux étant un signal périodique de fréquence $\frac{f}{2} = \frac{1}{2P_m}$.
- le déphasage φ dépend de la coordonnée x selon φ = φ$_0$ + a x
- la fonction de démodulation est exp(-$2\pi jft$)

on peut utiliser

- une fonction de marquage associé au point de coordonnées (x,z) proportionnelle à l'expression

$$M(x,z) = -jexp\left(\frac{-2\pi jfz}{u} + j\varphi_0 + jax\right)$$

- une composante de Fourrier $\tilde{S}(v_x, v_z)$ de l'image acousto-optique qui peut être déterminée en choisissant a = -$2\pi v_x$, f = $uv_z$ et à partir du calcul suivant :

$$\tilde{S}(v_x, v_z) = \Phi_d\left(+\frac{\pi}{2}, a, f\right)$$

Le dispositif de commande 7,17 peut être configuré pour générer, à une fréquence de modulation temporelle *f* donnée du signal de modulation temporelle, les différentes ondes ultrasonores correspondant aux différentes fonctions reliant le déphasage (φ) et la position du transducteur ultrasonore nécessaires pour déterminer la composante de Fourier $\tilde{S}(v_x, v_z)$ de l'image acousto-optique.

Il est à noter que l'utilisation du signal constant par morceaux tel qu'il a été défini plus haut permet de limiter certains phénomènes de diffraction spécifique en champ proche (comme l'effet de Moiré).

*Mesure et démodulation du signal acousto-optique avec trois fréquences spatiales*

Ce cas correspond à la situation dans laquelle a et b sont non nuls dans l'expression φ = φ$_0$ + a x + by.

Pour un système de détection de type interférométrique où :

- la porteuse du signal de commande est un signal carré de fréquence ultrasonore f$_{US}$,

- le signal de modulation temporelle est un signal carré de fréquence $f = \frac{1}{P_m}$ alternant entre les valeurs -1 et +1 à chaque demi-période temporelle $\frac{P_m}{2} = \frac{1}{2f}$

- le déphasage φ dépend des coordonnées x et y selon φ = φ$_0$ + a x + b y

- la fonction de démodulation est cos($2\pi f_{US} t + \psi$) * sin($2\pi f t$) où $\psi$ est une phase,

on peut utiliser

- une fonction de marquage associée au point de coordonnées (x, y, z) proportionnelle à l'expression

$$M(x, y, z) = \cos^2\left(2\frac{\pi f z}{u} - \varphi_0 - ax - by\right)$$
$$= \frac{1}{2}\left(\cos\left(2\pi\left(\frac{2f}{u}z - \frac{a}{\pi}x - \frac{b}{\pi}y\right) - 2\varphi_0\right) + 1\right)$$

- une composante de Fourrier $\tilde{S}(v_x, v_y, v_z)$ de l'image acousto-optique qui peut être déterminée en choisissant a = $-\pi v_x$, b = $-\pi v_y$, $f = \frac{u v_z}{2}$ et à partir du calcul suivant :

$$\tilde{S}(v_x, v_y, v_z) = \Phi_d{}^2(0, a, b, f) + j.\Phi_d{}^2\left(-\frac{\pi}{4}, a, b, f\right)$$
$$-\left(\Phi_d{}^2\left(+\frac{\pi}{2}, a, b, f\right) + j.\Phi_d{}^2\left(+\frac{\pi}{4}, a, b, f\right)\right)$$

- une moyenne des composantes de Fourier obtenues pour différentes valeurs de phase $\psi$, voire pour plusieurs acquisitions, toutes choses étant égales par ailleurs.

Pour un système de détection de type incohérent où :

- la porteuse du signal de commande est un signal carré de fréquence ultrasonore f$_{US}$,

- le signal de modulation temporelle est un signal carré de fréquence $f = \frac{1}{P_m}$ alternant entre les valeurs +1 et 0 à chaque demi-période temporelle $\frac{P_m}{2} = \frac{1}{2f}$, ou un signal constant par morceaux prenant alternativement au cours d'une période les valeurs +1, 0, -1, 0 durant quatre durées égales, le signal constant par morceaux étant un signal périodique de fréquence $\frac{f}{2} = \frac{1}{2P_m}$.

- le déphasage φ dépend des coordonnées x et y selon φ = φ$_0$ + a x + b y

- la fonction de démodulation est sin($2\pi f t$),

on peut utiliser

- une fonction de marquage associé au point de coordonnées (x, y, z) proportionnelle à l'expression

$$M(x, y, z) = \cos\left(\frac{2\pi f z}{u} - \varphi_0 - ax - by\right)$$

- une composante de Fourrier $\tilde{S}(v_x, v_y, v_z)$ de l'image acousto-optique qui peut être déterminée en choisissant a = $-2\pi v_x$, b = $-2\pi v_y$, $f = u v_z$ et à partir du calcul suivant :

$$\tilde{S}(v_x, v_y, v_z) = \Phi_d(0, a, b, f) + j.\Phi_d\left(-\frac{\pi}{2}, a, b, f\right)$$

Pour un système de détection de type incohérent où :

- la porteuse du signal de commande est un signal carré de fréquence ultrasonore $f_{US}$,

- le signal de modulation temporelle est un signal carré de fréquence $f = \frac{1}{P_m}$ alternant entre les valeurs 0 et +1 à chaque demi-période temporelle $\frac{P_m}{2} = \frac{1}{2f}$ ou un signal constant par morceaux prenant alternativement au cours d'une période les valeurs +1, 0, -1, 0 durant quatre durées égales, le signal constant par morceaux étant un signal périodique de fréquence $\frac{f}{2} = \frac{1}{2P_m}$.

- le déphasage $\varphi$ dépend des coordonnées x et y selon $\varphi = \varphi_0 + a\,x + b\,y$
- la fonction de démodulation est $\exp(-2\pi jft)$

on peut utiliser

- une fonction de marquage associé au point de coordonnées (x,y,z) proportionnelle à l'expression

$$M(\mathrm{x,y,z}) = -j exp\left(-\frac{2\pi jfz}{u} + j\varphi_0 + jax + jby\right)$$

- une composante de Fourrier $\tilde{S}(v_x, v_y, v_z)$ de l'image acousto-optique qui peut être déterminée en choisissant $a = -2\pi v_x$ , $b = -2\pi v_y$, $f = u v_z$ et à partir du calcul suivant :

$$\tilde{S}(v_x, v_y, v_z) = \Phi_d\left(+\frac{\pi}{2}, \mathrm{a, b,} f\right)$$

Le dispositif de commande 7,17 peut être configuré pour générer, à une fréquence de modulation temporelle $f$ donnée du signal de modulation temporelle, les différentes ondes ultrasonores correspondant aux différentes fonctions reliant le déphasage ($\varphi$) et la position du transducteur ultrasonore nécessaires pour déterminer la composante de Fourrier $\tilde{S}(v_x, v_y, v_z)$ de l'image acousto-optique.

Le dispositif de commande 7,17 peut être configuré en outre pour générer les différentes ondes ultrasonores correspondant à différentes valeurs de la fréquence temporelle $f$ donnée du signal de modulation temporelle de façon à déterminer avec le moyen de traitement 10,110 des composantes de Fourier d'une image acousto-optique de la zone d'observation, et à reconstruire de ladite image par transformation de Fourier inverse.

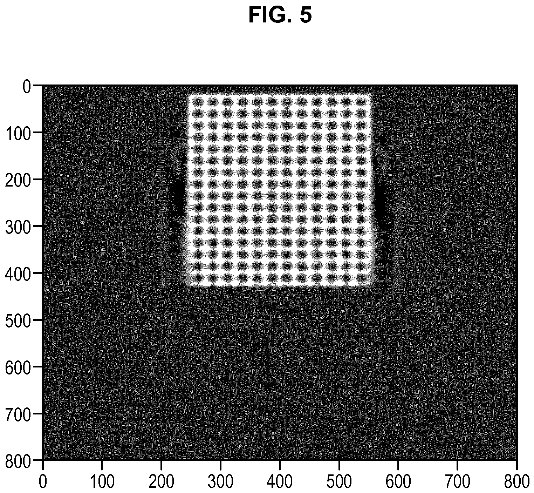

La figure 5 représente la simulation de l'image bidimensionnelle d'un objet obtenue par un système d'imagerie acousto-optique selon un mode de réalisation de l'invention.

L'objet est une grille dont la maille est de 2.5 millimètres.

12880 composantes de Fourier, correspondant à 161 valeurs de fréquence spatiale $v_x$ et 80 valeurs de fréquence spatiale $v_z$ , ont été calculées pour construire une image de la grille.

Pour chaque composante de Fourier, la propagation de l'onde ultrasonore correspondante est simulée, la fonction de marquage correspondante est déterminée en utilisant une démodulation sinusoïdale.

L'image obtenue fait apparaître un objet bien résolu, y compris à proximité des transducteurs.

Les zones en bordure de champ, à droite et à gauche de l'image, apparaissent atténuées, mais ne sont pas brouillées.

En dehors des bordures, la grille n'est pas déformée.

Le rapport signal sur bruit est à peu près constant sur l'ensemble de la grille.

La résolution ne se dégrade pas à partir du centre de l'image, il n'y a pas de limitation de l'extension spatiale de la zone observable.

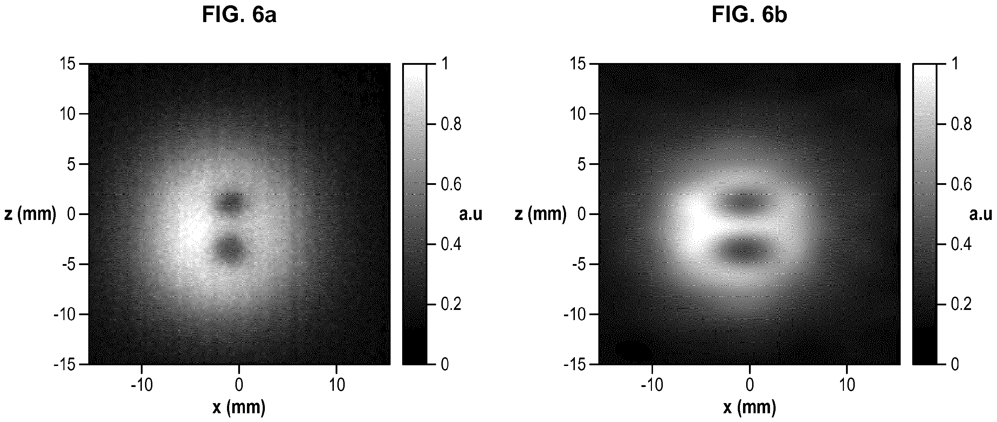

Les figures 6a et 6b représentent les images acousto-optiques d'un même objet qui est un échantillon diffusant de 2,5cm d'épaisseur, avec deux inclusions absorbantes de 3mm de diamètre et séparées de 3mm.

La figure 6a est obtenue par un système d'imagerie selon une technique standard d'ultrasons focalisés de l'art antérieur.

Pour chaque acquisition, quelques transducteurs voisins produisent de manière synchronisée 3 périodes d'une onde ultrasonore. Cela permet d'imager une partie de l'objet. Pour l'acquisition suivante, d'autres transducteurs sont utilisés. On forme ainsi peu à peu l'image de l'objet.

Relativement peu d'énergie est envoyée à chaque acquisition de sorte que le signal acousto-optique récupéré est relativement faible. Par ailleurs, l'onde est focalisée et l'intensité ultrasonore est relativement importante.

La figure 6b est obtenue par un système d'imagerie selon un mode de réalisation de l'invention. Il s'agit d'une détection incohérente par holographie adaptative. Pour chaque acquisition, l'ensemble des transducteurs est utilisé et une composante de la transformée de Fourier est déterminée.

Beaucoup plus d'énergie est envoyé vers le système de sorte que le signal acousto-optique récupéré est relativement important. De plus l'onde n'étant pas focalisée, l'intensité ultrasonore reste relativement faible.

Les inclusions apparaissent beaucoup plus contrastées sur la figure 6b. Le rapport signal sur bruit dans la figure 6b est donc bien plus important que dans la figure 6a, alors que l'intensité ultrasonore maximale est bien inférieure dans le cas de la figure 6b que dans le cas de la figure 6a.

La zone observable est plus étendue dans le cas de la figure 6b que dans le cas de la figure 6a.

*Exemple d'acquisition d'une image à deux dimensions. L'exemple 1 correspond au cas de la figure 5 ; l'exemple 2 au cas de la figure 6b.*

| Grandeur | Notation/Formule | Exemple 1 | Exemple 2 |
|---|---|---|---|
| Taille de la zone d'observation selon l'axe Dz | $L_z$ | 80mm | 30mm |
| Taille de la zone d'observation selon l'axe Dx (ou Dy) | $L_x$ (ou $L_y$) | 80mm | 38,4mm |
| Résolution spatiale suivant Dz - longueur du plus petit détail à imager selon Dz | $\Delta z$ | 0.5mm | 1,75mm |
| Résolution spatiale suivant Dx (ou Dy) - longueur du plus petit détail à imager selon Dx (ou Dy) | $\Delta x$ ($\Delta y$) | 0.5mm | 3,5mm |
| | | | |
| Fréquences spatiales $\nu_z$ | $\nu_z = k/L_z$ (*) avec k entier | $k \times 0{,}125\text{cm}^{-1}$ | $k \times 0{,}33\text{cm}^{-1}$ |
| Fréquence $\nu_z$ maximale | $1/(2\Delta z)$ | $10\text{cm}^{-1}$ | $2{,}85\text{cm}^{-1}$ |
| Nombre de fréquences $\nu_z$ différentes | $L_z /(2\Delta z)$ | 80 | 8 |
| | | | |
| Vitesse de propagation des ultrasons | u | 1500m/s | 1500m/s |
| Fréquence de modulation temporelle f | $f = u\nu_z = ku/L_z$ | $k \times 18{,}75\text{kHz}$ | $k \times 50\text{kHz}$ |
| Fréquence f maximale | $u/(2\Delta z)$ | 1500kHz | 400kHz |
| | | | |
| Fréquences spatiales $\nu_x$ (ou $\nu_y$) | $\nu_x = k/L_x$ (ou $\nu_y = k/L_y$) avec k entier | $k \times 0{,}125\text{cm}^{-1}$ | $k \times 0{,}26\text{cm}^{-1}$ |
| Fréquence $\nu_x$ (ou $\nu_y$) maximale | $|\nu_x| < 1/(2\Delta x)$ (ou $|\nu_y| < 1/(2\Delta y)$) | $10\text{cm}^{-1}$ | $1{,}43\text{cm}^{-1}$ |
| Nombre de fréquences $\nu_x$ (ou $\nu_y$) différentes | $1 + 2 L_x /(2\Delta x)$ (ou $1 + 2 L_y /(2\Delta y)$) | 161 | 11 |
| | | | |
| Nombre (**) de composantes de Fourier à déterminer | $L_x /(2\Delta x) \times L_z /(2\Delta z)$ (ou $L_y /(2\Delta y) \times L_z /(2\Delta z)$) | 12880 | 88 |
| (*)° On prendra $\nu_z$ et k>0 et on utilise : $S(-\nu_x, -\nu_y, -\nu_z) = S^*(\nu_x, \nu_y, \nu_z)$. La valeur $S(\nu_x, \nu_y, 0)$ est obtenue par extrapolation. (**) Le nombre de composantes de Fourier à déterminer est égal au produit du nombre de fréquences $\nu_z$ différentes à déterminer par le nombre de fréquences $\nu_x$ (ou $\nu_y$) différentes à déterminer. | | | |

Le nombre d'acquisitions dépend de la résolution spatiale et de la taille de l'image. Il est environ égal au produit des rapports pour les différentes directions de la taille de l'image dans une direction divisée par la résolution dans cette direction.

Plus la résolution est faible et plus le nombre d'acquisitions est important.

Plus la taille de l'image est importante et plus le nombre d'acquisitions est important.

**Revendications**

1. Système d'imagerie acousto-optique d'une zone d'observation (3, 13) d'un milieu, comprenant :

  • un ensemble de transducteurs ultrasonores (1, 11) alignés selon au moins une direction d'alignement (Dx, Dy), aptes à produire ensemble, une onde ultrasonore (2, 12) dans la zone d'observation (3, 13), se propageant selon une direction de propagation (Dz) orthogonale à la ou aux directions d'alignement (Dx, Dy) des transducteurs ultrasonores (1, 11);
  • une source lumineuse (4, 14) produisant une onde lumineuse incidente (5, 15), rencontrant ladite onde ultrasonore dans la zone d'observation pour produire des photons marqués (6, 16) ;
  • un dispositif de commande (7, 17) configuré pour piloter chaque transducteur ultrasonore (1, 11) avec un signal de commande de fréquence ultrasonore ($f_{US}$) ;
  • un système de détection (8, 18) d'une partie des photons marqués, lesdits photons marqués portant une modulation temporelle transmise par l'onde ultrasonore rencontrant l'onde lumineuse ;
  • un moyen de traitement (10, 110) d'un signal d'acquisition acousto-optique (9, 19) reçu du système de détection, apte à démoduler ledit signal d'acquisition acousto-optique ;

  **caractérisé en ce que** le dispositif de commande (7, 17) est configuré pour :

  - piloter chaque transducteur ultrasonore (1,11) par le signal de commande, ledit signal de commande étant constitué d'une onde porteuse de fréquence ultrasonore ($f_{US}$),

    l'onde porteuse étant identique pour tous les transducteurs de façon à générer une onde plane non focalisée se propageant dans la direction (Dz),
    cette onde plane non focalisée étant modulée par un signal de modulation temporelle périodique de fréquence temporelle donnée et de même forme pour tous les transducteurs ultrasonores (1, 11),
    ce signal de modulation temporelle ayant un déphasage ( φ) dont la valeur est spécifique à chaque transducteur ultrasonore et qui est une fonction de la position des transducteurs ultrasonores (1, 11),
    de sorte que l'onde ultrasonore plane émise par l'ensemble des transducteurs ultrasonores (1, 11) présente une modulation, qui a une périodicité spatiale selon la ou les directions d'alignement (Dx, Dy) des transducteurs dont la valeur est déterminée par la fonction entre le déphasage (cp) et la position des transducteurs ultrasonores,

  - générer différentes ondes ultrasonores planes non focalisées se propageant dans la même direction (Dz) orthogonale à la ou aux directions d'alignement (Dx, Dy) des transducteurs ultrasonores (1, 11), et modulées par un signal de modulation temporelle périodique de fréquence temporelle donnée et de même forme pour tous les transducteurs ultrasonores (1, 11),
    pour différentes valeurs de la fréquence temporelle donnée du signal de modulation temporelle,
    avec, pour chaque fréquence temporelle donnée, différentes fonctions reliant le déphasage (cp) et la position des transducteurs ultrasonores (1, 11), les différentes ondes ultrasonores correspondant aux différentes fonctions reliant le déphasage (cp) et la position du transducteur ultrasonore,

  pour que le moyen de traitement (10, 110) :

  • détermine des composantes de Fourier d'une image acousto-optique (21, 121) de la zone d'observation (3, 13), et
  • reconstruise spatialement ladite image acousto-optique (21, 121) par transformation de Fourier inverse des composantes de Fourier.

2. Système d'imagerie acousto-optique selon la revendication 1, **caractérisé en ce que** la position de chaque transducteur ultrasonore (1, 11) est définie par une première coordonnée (x) selon la première direction d'alignement (Dx) orthogonale à la direction de propagation (Dz) et une deuxième coordonnée (y) selon une deuxième direction d'alignement (Dy) orthogonale à la direction de propagation (Dz), le déphasage (cp) est une fonction affine des première et deuxième coordonnées (x, y) des transducteurs ultrasonores (1, 11) de la forme $\varphi = \varphi_0 + a\,x + b\,y$, où $\varphi_0$, a et b sont des paramètres définissant la relation affine, le choix de ces paramètres permettant de déterminer la période spatiale de la modulation de l'onde ultrasonore plane selon la ou les directions d'alignement des transducteurs (1, 11) orthogonales à la direction de propagation (Dz).

**3.** Système d'imagerie acousto-optique selon la revendication 2, **caractérisé en ce que** les transducteurs ultrasonores (1, 11) sont alignés et répartis uniquement selon la première direction d'alignement (Dx), le paramètre b étant nul, et **en ce que** l'onde ultrasonore plane se propage dans un plan d'imagerie défini par la direction de propagation (Dz) et la première direction d'alignement (Dx).

**4.** Système d'imagerie acousto-optique selon l'une des revendications 1 à 2, **caractérisé en ce que** les transducteurs ultrasonores (1, 11) forment un réseau dans le plan défini par les première et deuxième directions d'alignement (Dx, Dy), et **en ce que** l'onde ultrasonore plane se propage dans un volume d'imagerie défini par la direction de propagation (Dz) et les première et deuxième direction d'alignement (Dx, Dy).

**5.** Système d'imagerie acousto-optique selon l'une des revendications 1 à 4, **caractérisé en ce que** le signal de modulation temporelle est un signal carré ou sinusoïdal.

**6.** Système d'imagerie acousto-optique selon la revendication 5, **caractérisé en ce que** :

- la position de chaque transducteur ultrasonore est définie par une première coordonnée (x) selon une première direction d'alignement (Dx) orthogonale à la direction de propagation et une deuxième coordonnée (y) selon une deuxième direction d'alignement (Dy) orthogonale à la direction de propagation (Dz) et à la première direction d'alignement (Dx),
ce système d'imagerie permet la mesure de composantes de Fourier de l'image acousto-optique de la zone d'observation associées à une première fréquence spatiale ($v_x$) suivant la première direction d'alignement (Dx), à une deuxième fréquence spatiale ($v_y$) suivant la deuxième direction d'alignement (Dy), et une troisième fréquence spatiale ($v_z$) non nulle suivant la direction de propagation (Dz),
- le signal de modulation temporelle est un signal périodique en créneau, dont la fréquence temporelle (f) est égale au produit de la troisième fréquence spatiale ($v_z$) par la vitesse (u) de propagation de l'onde ultrasonore plane dans le milieu, alternant les valeurs +1 et 0 à chaque demi-période temporelle (1/(2f)),
- le déphasage ($\varphi$) est une fonction affine des première et deuxième coordonnées (x, y) des transducteurs ultrasonores de la forme $\varphi = \varphi_0 - 2\pi v_x x - 2\pi v_y Y$,
- le signal d'acquisition acousto-optique (9) reçu du système de détection est directement proportionnel à la puissance lumineuse des photons marqués détectés par le système de détection,
- le moyen de traitement multiplie le signal d'acquisition acousto-optique reçu du système de détection par une fonction de démodulation temporelle (20), intègre le résultat sur un intervalle de temps prédéterminé qui est un multiple de la période temporelle de la modulation temporelle (1/f), pour obtenir un signal démodulé,
- la composante de Fourier de l'image acousto-optique de la zone d'observation associée à une première, deuxième et troisième fréquences spatiales ($v_x$, $v_y$, $v_z$) est obtenue à partir d'un signal démodulé ou de plusieurs signaux démodulés correspondant à différentes valeurs du paramètre $\varphi_0$.

**7.** Système d'imagerie acousto-optique selon la revendication 6, **caractérisé en ce que** la fonction de démodulation temporelle est $\exp(-2\pi j f t)$, le signal démodulé étant alors un nombre complexe, et la composante de Fourier étant simplement proportionnelle au signal démodulé obtenu pour $\varphi_0 = \pi/2$.

**8.** Système d'imagerie acousto-optique selon la revendication 6, **caractérisé en ce que** la fonction de démodulation temporelle est $\sin(2\pi f t)$, la composante de Fourier étant alors proportionnelle à la somme du signal démodulé obtenu pour $\varphi_0 = 0$ et de j (nombre imaginaire) multiplié par le signal démodulé obtenu pour $\varphi_0 = -\pi/2$ .

**9.** Système d'imagerie acousto-optique selon la revendication 5, **caractérisé en ce que** :

- la position du transducteur ultrasonore est définie par une première coordonnée (x) selon une première direction d'alignement (Dx) orthogonale à la direction de propagation (Dz) et une deuxième coordonnée (y) selon une deuxième direction d'alignement (Dy) orthogonale à la direction de propagation (Dz) et à la première direction d'alignement (Dx),
- ce système d'imagerie permet la mesure de composantes de Fourier de l'image acousto-optique de la zone d'observation associées à une première fréquence spatiale ($v_x$) associée à la première direction d'alignement (Dx), à une deuxième fréquence spatiale ($v_y$) associée à la deuxième direction d'alignement (Dy), et une troisième fréquence spatiale ($v_z$) non nulle associée à la direction de propagation (Dz),
- le signal de modulation temporelle est un signal périodique en créneau, dont la fréquence temporelle (f) est égale à la moitié du produit de la fréquence spatiale $v_z$ par la vitesse (u) de propagation de l'onde ultrasonore dans le milieu, alternant les valeurs -1 et +1 à chaque demi-période temporelle (1/(2f)),

- le déphasage (φ) est une fonction affine des première et deuxième coordonnées (x, y) des transducteurs ultrasonores de la forme $\varphi = \varphi_0 - \pi \nu_x x - \pi \nu_y y$,
- le signal d'acquisition acousto-optique (19) reçu du système de détection est un signal interférométrique entre des photons marqués détectés par le système de détection et une onde lumineuse de référence (122) de même longueur d'onde que l'onde lumineuse incidente dans la zone d'observation,
- le moyen de traitement multiplie le signal d'acquisition acousto-optique reçu du système de détection par une fonction de démodulation temporelle (120), intègre le résultat sur un intervalle de temps prédéterminé qui est un multiple de la demi-période temporelle de la modulation temporelle (1/(2f)), pour obtenir un signal démodulé,
- le signal démodulé est multiplié par lui-même, ou par un signal démodulé enregistré antérieurement dans des conditions identiques, pour obtenir un signal démodulé élevé au carré, qui est une quantité proportionnelle à l'énergie lumineuse des photons marqués détectés par le système de détection pendant l'intervalle de temps prédéterminé,
- la composante de Fourier de l'image acousto-optique de la zone d'observation associée à une première, deuxième et troisième fréquences spatiales ($\nu_x$, $\nu_y$, $\nu_z$) est obtenue à partir de plusieurs signaux démodulés élevés au carré et correspondants à différentes valeurs du paramètre $\varphi_0$.

10. Système d'imagerie acousto-optique selon la revendication 9, **caractérisé en ce que** la fonction de démodulation temporelle est $\sin(2\pi f t)$ multiplié par $\cos(2\pi \text{fus } t+\psi)$, où fus est la fréquence de l'onde porteuse du signal de commande de fréquence ultrasonore et $\psi$ une phase, ladite composante de Fourier étant alors proportionnelle à la somme du signal démodulé élevé au carré obtenu pour $\varphi_0=0$ et de j (nombre imaginaire) multiplié par le signal démodulé élevé au carré obtenu pour $\varphi_0=-\pi/4$, auquel on aura retranché la somme du signal démodulé élevé au carré obtenu pour $\varphi_0=\pi/2$ et de j (nombre imaginaire) multiplié par le signal démodulé élevé au carré obtenu pour $\varphi_0=+\pi/4$.

11. Système d'imagerie acousto-optique selon la revendication 10, **caractérisé en ce que** la reconstruction de l'image par transformation de Fourier inverse est effectuée en utilisant une moyenne de composantes de Fourier obtenues pour différentes valeurs de phase $\psi$, toutes choses étant égales par ailleurs.

12. Procédé d'imagerie acousto-optique d'une zone d'observation (3, 13) d'un milieu, le procédé comprenant :

   • E1 : une étape de génération d'une onde lumineuse (5, 15) incidente dans la zone d'observation,
   • E2 : une étape de génération par un ensemble de transducteurs ultrasonores (1, 11) alignés selon au moins une direction d'alignement, d'une onde ultrasonore (2, 12) dans la zone d'observation, avec une fréquence ultrasonore (fus), se propageant selon une direction de propagation (Dz) orthogonale à l'au moins ou aux directions d'alignement des transducteurs ultrasonores (1, 11),
   • E3 : une étape de génération de photons marqués (6, 16) et portant une modulation temporelle transmise par l'onde ultrasonore rencontrant l'onde lumineuse,
   • E4 : une étape de détection d'une partie des photons marqués et de génération d'un signal d'acquisition acousto-optique (9, 19) par un système de détection (8, 18),
   • E5 : une étape de traitement dudit signal d'acquisition acousto-optique fourni par ledit système de détection comprenant une étape de démodulation temporelle dudit signal d'acquisition acousto-optique en utilisant un signal de démodulation temporelle (20, 120),

   **caractérisé en ce que** :

   (i) dans l'étape de génération (E2) tous les transducteurs ultrasonores (1, 11) sont pilotés par un signal de commande constitué d'une onde porteuse de fréquence ultrasonore (fus),

      l'onde porteuse étant identique pour tous les transducteurs ultrasonores (1, 11), de façon à générer une onde plane non focalisée se propageant dans la direction (Dz) orthogonale à la ou aux directions d'alignement (Dx, Dy) des transducteurs ultrasonores (1, 11),
      cette onde plane non focalisée étant modulée par un signal de modulation temporelle périodique de fréquence temporelle donnée et de même forme pour tous les transducteurs ultrasonores (1, 11),
      ce signal de modulation temporelle ayant un déphasage (cp) dont la valeur est spécifique à chaque transducteur ultrasonore et qui est une fonction de la position des transducteurs ultrasonores (1, 11),
      de sorte que l'onde ultrasonore plane émise par l'ensemble des transducteurs ultrasonores présente une modulation, qui a une périodicité spatiale selon la ou les directions d'alignement des transducteurs dont la valeur de la période spatiale est déterminée par la fonction entre le déphasage (cp) et la position des transducteurs ultrasonores (1, 11);

(ii) le procédé comprenant en outre une étape E6 où les étapes précédentes E1 à E5 sont répétées plusieurs fois, pour différentes valeurs de la fréquence temporelle donnée du signal de modulation temporelle,

avec, pour chaque fréquence temporelle donnée, différentes fonctions reliant le déphasage ($\varphi$) et la position des transducteurs ultrasonores (1, 11), les différentes ondes ultrasonores non focalisées se propageant dans la même direction (Dz) orthogonale à la ou aux directions d'alignement (Dx, Dy) des transducteurs ultrasonores (1, 11), correspondant aux différentes fonctions reliant le déphasage (cp) et la position du transducteur ultrasonore,

de façon à déterminer des composantes de Fourier d'une image acousto-optique (21, 121) de la zone d'observation (3, 13), et à reconstruire spatialement ladite image acousto-optique (21, 121) par transformation de Fourier inverse des composantes de Fourier.

13. Procédé d'imagerie acousto-optique selon la revendication 12, **caractérisé en ce que** la position de chaque transducteur ultrasonore (1, 11) est définie par une première coordonnée (x) selon une première direction d'alignement (Dx) orthogonale à la direction de propagation (Dz) et une deuxième coordonnée (y) selon une deuxième direction d'alignement (Dy) orthogonale à la direction de propagation (Dz) et à la première direction d'alignement (Dx), le déphasage ($\varphi$) est une fonction affine des première et deuxième coordonnées (x, y) des transducteurs ultrasonores (1, 11) : $\varphi = \varphi_0 + a x + b y$, où $\varphi_0$, a et b sont des paramètres définissant la relation affine, le choix de ces paramètres permettant de déterminer la période spatiale de la modulation de l'onde ultrasonore selon la ou les directions d'alignement orthogonales à la direction de propagation (Dz).

14. Procédé d'imagerie acousto-optique selon l'une des revendications 12 à 13, **caractérisé en ce que** les transducteurs ultrasonores (1, 11) sont alignés et uniquement répartis selon la première direction d'alignement (Dx), respectivement la deuxième direction d'alignement (Dy), le paramètre b, respectivement a, étant nul, et **en ce que** l'onde ultrasonore plane se propage dans un plan d'imagerie défini par la direction de propagation (Dz) et la première direction d'alignement (Dx), respectivement la deuxième direction d'alignement (Dy).

15. Procédé d'imagerie acousto-optique selon l'une des revendications 12 à 13, **caractérisé en ce que** les transducteurs ultrasonores (1, 11) forment un réseau dans le plan défini par les première et deuxième directions d'alignement (Dx, Dy) et **en ce que** l'onde ultrasonore plane se propage dans un volume d'imagerie défini par la direction de propagation (Dz) et les première et deuxième direction d'alignement (Dx, Dy).

16. Procédé d'imagerie acousto-optique selon l'une des revendications 12 à 15, **caractérisé en ce que** le signal de modulation temporelle est un signal carré ou un signal sinusoïdal.

17. Procédé d'imagerie acousto-optique selon la revendication 16, **caractérisé en ce que** :

- le procédé d'imagerie permet la mesure de composantes de Fourier de l'image acousto-optique de la zone d'observation associées à une première fréquence spatiale ($v_x$) suivant la première direction d'alignement (Dx), à une deuxième fréquence spatiale ($v_y$) suivant la deuxième direction d'alignement (Dy), et une troisième fréquence spatiale ($v_z$) non nulle suivant la direction de propagation (Dz),
- le signal de modulation temporelle est un signal périodique en créneau, dont la fréquence temporelle (f) est égale au produit de la fréquence spatiale $v_z$ par la vitesse (u) de propagation de l'onde ultrasonore plane dans le milieu, alternant les valeurs +1 et 0 à chaque demi-période temporelle (1/(2f)),
- le déphasage ($\varphi$) est une fonction affine des première et deuxième coordonnées (x, y) des transducteurs ultrasonores ($\varphi = \varphi_0 - 2\pi\, v_x x - 2\pi\, v_y y$),
- le signal d'acquisition acousto-optique (9) issu de l'étape de détection est directement proportionnel à la puissance lumineuse des photons marqués détectés à l'étape de détection,
- l'étape de traitement consiste à multiplier le signal d'acquisition acousto-optique issu de l'étape de détection par une fonction de démodulation temporelle, et à intégrer le résultat sur un intervalle de temps prédéterminé qui est un multiple de la période de modulation temporelle (1/f), pour obtenir un signal démodulé,
- la composante de Fourier de l'image acousto-optique de la zone d'observation associée à une première, deuxième et troisième fréquences spatiales ($v_x$, $v_y$, $v_z$) est obtenue à partir d'un signal démodulé ou de plusieurs signaux démodulés correspondant à différentes valeurs du paramètre $\varphi_0$.

18. Procédé d'imagerie acousto-optique selon la revendication 17, **caractérisé en ce que** la fonction de démodulation temporelle est $\exp(-2\pi\, j\, f\, t)$, le signal démodulé étant alors un nombre complexe, et la composante de Fourier étant simplement proportionnelle au signal démodulé obtenu pour $\varphi_0 = \pi/2$.

**19.** Procédé d'imagerie acousto-optique selon la revendication 17, **caractérisé en ce que** la fonction de démodulation temporelle est $\sin(2\pi f t)$, la composante de Fourier étant alors proportionnelle à la somme du signal démodulé obtenu pour $\varphi_0=0$ et de j (nombre imaginaire) multiplié par le signal démodulé obtenu pour $\varphi_0=-\pi/2$ .

**20.** Procédé d'imagerie acousto-optique selon la revendication 16, **caractérisé en ce que** :

- la position de chaque transducteur ultrasonore (1, 11) est définie par une première coordonnée (x) selon une première direction d'alignement (Dx) orthogonale à la direction de propagation (Dz) et une deuxième coordonnée (y) selon une deuxième direction (Dy) d'alignement orthogonale à la direction de propagation (Dz) et à la première direction d'alignement (Dx),
- le procédé d'imagerie permet la mesure de composantes de Fourier de l'image acousto-optique de la zone d'observation associées à une première fréquence spatiale ($v_x$) suivant la première direction d'alignement (Dx), à une deuxième fréquence spatiale ($v_y$) suivant la deuxième direction d'alignement (Dy), et une troisième fréquence spatiale ($v_z$) non nulle suivant la direction de propagation (Dz),
- le signal de modulation temporelle est un signal périodique en créneau, dont la fréquence temporelle (f) est égale à la moitié du produit de la fréquence spatiale $v_z$ par la vitesse (u) de propagation de l'onde ultra-sonore dans le milieu, alternant les valeurs -1 et +1 à chaque demi-période temporelle (1/(2f)),
- le déphasage (cp) est une fonction affine des première et deuxième coordonnées (x, y) des transducteurs ultrasonores ($\varphi = \varphi_0 - \pi v_x x - \pi v_y y$),
- le signal d'acquisition acousto-optique (19) issu de l'étape de détection est un signal interférométrique entre des photons marqués détectés à l'étape de détection et une onde lumineuse de référence de même longueur d'onde que l'onde lumineuse incidente dans la zone d'observation,
- l'étape de traitement consiste à multiplier le signal d'acquisition acousto-optique issu de l'étape de détection par une fonction de démodulation temporelle, et à intégrer le résultat sur un intervalle de temps prédéterminé qui est un multiple de la demi-période temporelle de modulation temporelle (1/(2f)), pour obtenir un signal démodulé,
- le signal démodulé est multiplié par lui-même, ou par un signal démodulé enregistré antérieurement dans des conditions identiques, pour obtenir un signal démodulé élevé au carré, qui est une quantité proportionnelle à l'énergie lumineuse des photons marqués détectés à l'étape de détection pendant l'intervalle de temps prédéterminé,
- la composante de Fourier de l'image acousto-optique de la zone d'observation associée à une première, deuxième et troisième fréquences spatiales ($v_x$, $v_y$, $v_z$) est obtenue par combinaison linéaire des signaux démodulés élevés au carré et correspondants à différentes valeurs du paramètre $\varphi_0$.

**21.** Procédé d'imagerie acousto-optique selon la revendication 20, **caractérisé en ce que** la fonction de démodulation temporelle est $\sin(2\pi f t)$ multiplié par $\cos(2\pi f_{US} t+\psi)$, où $f_{US}$ est la fréquence de l'onde porteuse du signal de commande de fréquence ultrasonore et $\psi$ un déphasage, la composante de Fourier étant alors proportionnelle à la somme du signal démodulé élevé au carré obtenu pour $\varphi_0=0$ et de j (nombre imaginaire) multiplié par le signal démodulé élevé au carré obtenu pour $\varphi_0=-\pi/4$ , auquel on aura retranché la somme du signal démodulé élevé au carré obtenu pour $\varphi_0=+\pi/2$ et de j (nombre imaginaire) multiplié par le signal démodulé élevé au carré obtenu pour $\varphi_0=+\pi/4$.

**22.** Procédé d'imagerie acousto-optique selon la revendication 21, **caractérisé en ce que** la reconstruction de l'image par transformation de Fourier inverse est effectuée en utilisant une moyenne de composantes de Fourier obtenues pour différentes valeurs du déphasage $\psi$, toutes choses étant égales par ailleurs.

**Patentansprüche**

**1.** Akusto-optisches Bildgebungssystem eines Beobachtungsbereichs (3, 13) eines Mediums, umfassend:

- eine Reihe von Ultraschallwandlern (1, 11), die in mindestens einer Ausrichtungsrichtung (Dx, Dy) ausgerichtet sind und zusammen in der Lage sind, eine Ultraschallwelle (2, 12) im Beobachtungsbereich (3, 13) zu erzeugen, die sich in einer Ausbreitungsrichtung (Dz) ausbreitet, die orthogonal zu der oder den Ausrichtungsrichtungen (Dx, Dy) der Ultraschallwandler (1, 11) ist;
- eine Lichtquelle (4, 14), die eine einfallende Lichtwelle (5, 15) erzeugt, welche auf die Ultraschallwelle im Beobachtungsbereich trifft, um markierte Photonen (6, 16) zu erzeugen;
- eine Steuereinrichtung (7, 17), die so eingerichtet ist, dass sie jeden Ultraschallwandler (1, 11) mit einem Ultraschallfrequenz-Steuersignal (fréquence ultrasonore - fus) steuert;
- ein Detektionssystem (8, 18) eines Teils der markierten Photonen, wobei die markierten Photonen eine zeitliche Modulation aufweisen, die von der Ultraschallwelle übertragen wird, auf welche die Lichtwelle trifft;
- ein Mittel (10, 110) zur Verarbeitung eines vom Detektionssystem empfangenen akusto-optischen Erfassungssignals (9, 19), das geeignet ist, das akusto-optische Erfassungssignal zu demodulieren;
**dadurch gekennzeichnet, dass** die Steuereinrichtung (7, 17) eingerichtet ist, um:

- jeden Ultraschallwandler (1, 11) durch das Steuersignal zu steuern, wobei das Steuersignal aus einer Trägerwelle mit Ultraschallfrequenz ($f_{US}$) besteht, wobei die Trägerwelle für alle Wandler identisch ist, so dass eine unfokussierte ebene Welle erzeugt wird, die sich in die Richtung (Dz) ausbreitet, wobei diese unfokussierte ebene Welle durch ein periodisches, zeitlich moduliertes Signal mit einer bestimmten zeitlichen Frequenz und gleicher Form für alle Ultraschallwandler (1, 11) moduliert wird, wobei dieses zeitlich modulierte Signal eine Phasenverschiebung ($\varphi$) aufweist, deren Wert spezifisch für jeden Ultraschallwandler ist und die eine Funktion der Position der Ultraschallwandler (1, 11) ist, so dass die von der Reihe von Ultraschallwandlern (1, 11) ausgestrahlte ebene Ultraschallwelle eine Modulation aufweist, die eine räumliche Periodizität in der bzw. den Ausrichtungsrichtung(en) (Dx, Dy) der Wandler hat, deren Wert durch die funktionale Abhängigkeit zwischen der Phasenverschiebung ($\varphi$) und der Position der Ultraschallwandler bestimmt wird,
- verschiedene, unfokussierte, ebene Ultraschallwellen zu erzeugen, die sich in derselben Richtung (Dz) orthogonal zu der bzw. zu den Ausrichtungsrichtungen (Dx, Dy) der Ultraschallwandler (1, 11) ausbreiten und durch ein periodisches zeitlich moduliertes Signal mit einer bestimmten zeitlichen Frequenz und gleicher Form für alle Ultraschallwandler (1, 11) für verschiedene Werte der gegebenen zeitlichen Frequenz des zeitlich modulierten Signals moduliert werden, wobei es für jede gegebene zeitliche Frequenz verschiedene funktionale Abhängigkeiten gibt, die die Phasenverschiebung ($\varphi$) und die Position der Ultraschallwandler (1, 11) verbinden, wobei die verschiedenen Ultraschallwellen den verschiedenen funktionalen Abhängigkeiten entsprechen, die die Phasenverschiebung ($\varphi$) und die Position des Ultraschallwandlers verbinden,

damit das Verarbeitungsmittel (10, 110):

- Fourier-Komponenten eines akusto-optischen Bildes (21, 121) des Beobachtungsbereichs (3, 13) bestimmt, und
- das akusto-optische Bild (21, 121) räumlich durch Fourier-Umkehrtransformation der Fourier-Komponenten rekonstruiert.

2. Akusto-optisches Bildgebungssystem nach Anspruch 1, **dadurch gekennzeichnet, dass** die Position jedes Ultraschallwandlers (1, 11) durch eine erste Koordinate (x) in der ersten orthogonal zur Ausbreitungsrichtung (Dz) liegenden Ausrichtungsrichtung (Dx) und durch eine zweite Koordinate (y) in einer zweiten orthogonal zur Ausbreitungsrichtung (Dz) liegenden Ausrichtungsrichtung (Dy) definiert ist, wobei die Phasenverschiebung ($\varphi$) eine affine Funktion der ersten und der zweiten Koordinate (x, y) der Ultraschallwandler (1, 11) in der Form $\varphi = \varphi_0 + a\,x + b\,y$ ist, wobei $\varphi_0$, a und b Parameter sind, die das affine Verhältnis definieren, wobei die Wahl dieser Parameter die Bestimmung der räumlichen Periode der Modulation der ebenen Ultraschallwelle in der bzw. den orthogonal zur Ausbreitungsrichtung (Dz) liegenden Ausrichtungsrichtung(en) der Wandler (1, 11) ermöglicht.

3. Akusto-optisches Bildgebungssystem nach Anspruch 2, **dadurch gekennzeichnet, dass** die Ultraschallwandler (1, 11) nur nach der ersten Ausrichtungsrichtung (Dx) ausgerichtet und verteilt sind, wobei der Parameter b null ist, und dass sich die ebene Ultraschallwelle in einer durch die Ausbreitungsrichtung (Dz) und die erste Ausrichtungsrichtung (Dx) definierten Bildgebungsebene ausbreitet.

4. Akusto-optisches Bildgebungssystem nach einem der Ansprüche 1 bis 2, **dadurch gekennzeichnet, dass** die Ultraschallwandler (1, 11) ein Netz in der durch die erste und zweite Ausrichtungsrichtung (Dx, Dy) definierten Ebene bilden und dass sich die ebene Ultraschallwelle in einem durch die Ausbreitungsrichtung (Dz) und die erste und zweite Ausrichtungsrichtung (Dx, Dy) definierten Bildgebungsvolumen ausbreitet.

5. Akusto-optisches Bildgebungssystem nach einem der Ansprüche 1 bis 4, **dadurch gekennzeichnet, dass** das zeitlich modulierte Signal ein Rechteck- oder Sinussignal ist.

6. Akusto-optisches Bildgebungssystem nach Anspruch 5, **dadurch gekennzeichnet, dass**:

- die Position jedes Ultraschallwandlers durch eine erste Koordinate (x) in einer ersten orthogonal zur Ausbreitungsrichtung liegenden Ausrichtungsrichtung (Dx) und einer zweiten Koordinate (y) in einer zweiten orthogonal zur Ausbreitungsrichtung (Dz) und zur ersten Ausrichtungsrichtung (Dx) liegenden Ausrichtungsrichtung (Dy) definiert wird,

dieses Bildgebungssystem ermöglicht die Messung von Fourier-Komponenten des akusto-optischen Bildes des Beobachtungsbereichs, die einer ersten räumlichen Frequenz ($v_x$) in der ersten Ausrichtungsrichtung (Dx), einer zweiten räumlichen Frequenz ($v_y$) in der zweiten Ausrichtungsrichtung (Dy) und einer dritten räumlichen Frequenz ($v_z$) in der Ausbreitungsrichtung (Dz) zugeordnet sind,

- das zeitlich modulierte Signal ein periodisches Rechteckwellensignal ist, dessen zeitliche Frequenz (f) gleich dem Produkt aus der dritten räumlichen Frequenz ($v_z$) und der Ausbreitungsgeschwindigkeit (u) der ebenen Ultraschallwelle im Medium ist, wobei sich die Werte +1 und 0 bei jeder halben zeitlichen Periode (1/(2f)) abwechseln,

- die Phasenverschiebung ($\varphi$) eine affine Funktion der ersten und zweiten Koordinaten (x, y) der Ultraschallwandler in der Form $\varphi = \varphi_0 - 2n\, v_x\, x - 2n\, v_y\, y$ ist,

- das vom Detektionssystem empfangene akusto-optische Erfassungssignal (9) direkt proportional zur Lichtleistung der markierten Photonen ist, die vom Detektionssystem erkannt werden,

- das Verarbeitungsmittel das vom Detektionssystem empfangene akusto-optische Erfassungssignal mit einer zeitlichen Demodulationsfunktion (20) multipliziert und das Ergebnis in ein vorbestimmtes Zeitintervall integriert, das ein Vielfaches der zeitlichen Periode der zeitlichen Modulation (1/f) ist, um ein demoduliertes Signal zu erhalten,

- die Fourier-Komponente des akusto-optischen Bildes des Beobachtungsbereichs, die einer ersten, zweiten und dritten räumlichen Frequenz ($v_x$, $v_y$, $v_z$) zugeordnet ist, aus einem demodulierten Signal oder mehreren demodulierten Signalen erhalten wird, die verschiedenen Werten des Parameters $\varphi_0$ entsprechen.

7. Akusto-optisches Bildgebungssystem nach Anspruch 6, **dadurch gekennzeichnet, dass** die zeitliche Demodulationsfunktion $\exp(-2n\, j\, f\, t)$ ist, wobei das demodulierte Signal dann eine komplexe Zahl ist, und die Fourier-Komponente einfach proportional zum demodulierten Signal ist, das für $\varphi_0 = \pi/2$ erhalten wird.

8. Akusto-optisches Bildgebungssystem nach Anspruch 6, **dadurch gekennzeichnet, dass** die zeitliche Demodulationsfunktion $\sin(2\pi\, f\, t)$ ist, wobei die Fourier-Komponente dann proportional zur Summe aus dem für $\varphi_0 = 0$ erhaltenen demodulierten Signal und aus j (imaginäre Zahl) multipliziert mit dem für $\varphi_0 = -\pi/2$ erhaltenen demodulierten Signal ist.

9. Akusto-optisches Bildgebungssystem nach Anspruch 5, **dadurch gekennzeichnet, dass**:

- die Position des Ultraschallwandlers durch eine erste Koordinate (x) in einer ersten orthogonal zur Ausbreitungsrichtung (Dz) liegenden Ausrichtungsrichtung (Dx) und einer zweiten Koordinate (y) in einer zweiten orthogonal zur Ausbreitungsrichtung (Dz) und zur ersten Ausrichtungsrichtung (Dx) liegenden Ausrichtungsrichtung (Dy) definiert wird,

- dieses Bildgebungssystem die Messung von Fourier-Komponenten des akusto-optischen Bildes des Beobachtungsbereichs ermöglicht, die mit einer ersten räumlichen Frequenz ($v_x$) in Verbindung mit der ersten Ausrichtungsrichtung (Dx), mit einer zweiten räumlichen Frequenz ($v_y$) in Verbindung mit der zweiten Ausrichtungsrichtung (Dy) und mit einer dritten räumlichen Frequenz ($v_z$) nicht null in Verbindung mit der Ausbreitungsrichtung (Dz) verbunden sind,

- das zeitlich modulierte Signal ein periodisches Rechteckwellensignal ist, dessen zeitliche Frequenz (f) gleich der Hälfte des Produkts aus der räumlichen Frequenz $v_z$ und der Ausbreitungsgeschwindigkeit (u) der Ultraschallwelle im Medium ist, wobei sich die Werte -1 und +1 bei jeder halben zeitlichen Periode (1/(2f)) abwechseln,

- die Phasenverschiebung ($\varphi$) eine affine Funktion der ersten und zweiten Koordinaten (x, y) der Ultraschallwandler in der Form $\varphi = \varphi_0 - \pi\, v_x\, x - \pi\, v_y\, y$ ist.

- das vom Detektionssystem empfangene akusto-optische Erfassungssignal (19) ein interferometrisches Signal zwischen markierten Photonen ist, die vom Detektionssystem erkannt werden, und einer Referenzlichtwelle (122) derselben Wellenlänge wie die in den Beobachtungsbereich einfallende Lichtwelle,

- das Verarbeitungsmittel das vom Detektionssystem empfangene akusto-optische Erfassungssignal mit einer zeitlichen Demodulationsfunktion (120) multipliziert und das Ergebnis in ein vorbestimmtes Zeitintervall integriert, das ein Vielfaches der halben zeitlichen Periode der zeitlichen Modulation (1/f) ist, um ein demoduliertes Signal zu erhalten,

- das demodulierte Signal mit sich selbst oder mit einem zuvor unter identischen Bedingungen aufgezeichneten demodulierten Signal multipliziert wird, um ein quadriertes demoduliertes Signal zu erhalten, das eine Menge proportional zur Lichtenergie der vom Detektionssystem während des vorbestimmten Zeitintervalls erkannten markierten Photonen ist,

- die Fourier-Komponente des akusto-optischen Bildes des Beobachtungsbereichs, die einer ersten, zweiten und

dritten räumlichen Frequenz ($v_x$, $v_y$, $v_z$) zugeordnet ist, aus mehreren quadrierten demodulierten Signalen, die verschiedenen Werten des Parameters $\varphi_0$ entsprechen, erhalten wird.

10. Akusto-optisches Bildgebungssystem nach Anspruch 9, **dadurch gekennzeichnet, dass** die zeitliche Demodulationsfunktion sin($2\pi$ f t) multipliziert mit cos($2\pi$ fus t+$\psi$) ist, wobei fus die Frequenz der Trägerwelle des Ultraschallfrequenz-Steuersignals und $\psi$ eine Phase ist, wobei die Fourier-Komponente dann proportional zur Summe aus dem für $\varphi_0$=0 erhaltenen demodulierten quadrierten Signal und aus j (imaginäre Zahl) multipliziert mit dem für $\varphi_0$ =-n/4 erhaltenen demodulierten quadrierten Signal ist, von dem die Summe aus dem für $\varphi_0$=$\pi$/2 erhaltenen quadrierten demodulierten Signal und von j (imaginäre Zahl) multipliziert mit dem für $\varphi_0$ =+n/4 erhaltenen quadratierten demodulierten Signal abgezogen wird.

11. Akusto-optisches Bildgebungssystem nach Anspruch 10, **dadurch gekennzeichnet, dass** die Bildrekonstruktion durch Fourier-Umkehrtransformation unter Verwendung eines Durchschnitts von Fourier-Komponenten durchgeführt wird, die für verschiedene y-Phasenwerte erhalten wurden, wobei ansonsten alles andere gleich bleibt.

12. Akusto-optisches Bildgebungsverfahren eines Beobachtungsbereichs (3, 13) eines Mediums, wobei das Verfahren umfasst:

- E1: einen Schritt zur Erzeugung einer Lichtwelle (5, 15), die in den Beobachtungsbereich einfällt,
- E2: einen Schritt zur Erzeugung einer Ultraschallwelle (2, 12) im Beobachtungsbereich durch eine Reihe von in mindestens einer Ausrichtungsrichtung ausgerichteten Ultraschallwandlern (1, 11), mit einer Ultraschallfrequenz (fréquence ultrasonore - fus), die sich in einer Ausbreitungsrichtung (Dz) ausbreitet, die orthogonal zu mindestens oder zu den Ausrichtungsrichtungen der Ultraschallwandler (1, 11) ist,
- E3: einen Schritt zur Erzeugung von markierten Photonen (6, 16) und mit einer zeitlichen Modulation, die von der Ultraschallwelle übertragen wird, die auf die Lichtwelle trifft,
- E4: einen Schritt zur Detektion eines Teils der markierten Photonen und zur Erzeugung eines akusto-optischen Erfassungssignals (9, 19) durch ein Detektionssystem (8, 18),
- E5: einen Schritt zur Verarbeitung des akusto-optischen Erfassungssignals, das von dem Detektionssystem bereitgestellt wird, umfassend einen Schritt zur zeitlichen Demodulation des akusto-optischen Erfassungssignals unter Verwendung eines zeitlichen Demodulationssignals (20, 120),

**dadurch gekennzeichnet, dass**:

(i) im Erzeugungsschritt (E2) alle Ultraschallwandler (1, 11) durch ein Steuersignal gesteuert werden, das aus einer Trägerwelle mit Ultraschallfrequenz ($f_{US}$) besteht, wobei die Trägerwelle für alle Ultraschallwandler (1, 11) identisch ist, so dass eine unfokussierte ebene Welle erzeugt wird, die sich in der Richtung (Dz) orthogonal zu der bzw. den Ausrichtungsrichtungen (Dx, Dy) der Ultraschallwandler (1, 11) ausbreitet, wobei diese unfokussierte ebene Welle durch ein periodisches, zeitlich moduliertes Signal mit einer bestimmten zeitlichen Frequenz und gleicher Form für alle Ultraschallwandler (1, 11) moduliert wird, wobei dieses zeitlich modulierte Signal eine Phasenverschiebung ($\varphi$) aufweist, deren Wert spezifisch für jeden Ultraschallwandler ist und die eine Funktion der Position der Ultraschallwandler (1, 11) ist, so dass die von allen Ultraschallwandlern ausgestrahlte ebene Ultraschallwelle eine Modulation aufweist, die eine räumliche Periodizität in der bzw. den Ausrichtungsrichtung(en) der Wandler hat, deren Wert für die räumliche Periode durch die funktionale Abhängigkeit zwischen der Phasenverschiebung ($\varphi$) und der Position der Ultraschallwandler (1, 11) bestimmt wird;
(ii) Verfahren, ferner umfassend einen Schritt E6, wobei die vorangegangenen Schritte E1 bis E5 mehrmals für verschiedene Werte der gegebenen zeitlichen Frequenz des zeitlich modulierten Signals wiederholt werden, mit verschiedenen die Phasenverschiebung ($\varphi$) und die Position der Ultraschallwandler (1, 11) verbindenden funktionalen Abhängigkeiten bei jeder gegebenen zeitlichen Frequenz, wobei sich die verschiedenen unfokussierten Ultraschallwellen in derselben Richtung (Dz) orthogonal zu der oder den Ausrichtungsrichtungen (Dx, Dy) der Ultraschallwandler (1, 11) ausbreiten, entsprechend den verschiedenen Funktionen, die die Phasenverschiebung ($\varphi$) und die Position des Ultraschallwandlers verbinden, um Fourier-Komponenten eines akusto-optischen Bildes (21, 121) des Beobachtungsbereichs (3, 13) zu bestimmen und das akusto-optische Bild (21, 121) durch Fourier-Umkehrtransformation der Fourier-Komponenten räumlich zu rekonstruieren.

13. Akusto-optisches Bildgebungsverfahren nach Anspruch 12, **dadurch gekennzeichnet, dass** die Position jedes Ultraschallwandlers (1, 11) durch eine erste Koordinate (x) in einer ersten orthogonal zur Ausbreitungsrichtung (Dz) liegenden Ausrichtungsrichtung (Dx) und einer zweiten Koordinate (y) in einer zweiten orthogonal zur Ausbreitungsrichtung (Dz) und zur ersten Ausrichtungsrichtung (Dx) liegenden Ausrichtungsrichtung (Dy) definiert ist, wobei die

Phasenverschiebung ($\varphi$) eine affine Funktion der ersten und der zweiten Koordinate (x, y) der Ultraschallwandler (1, 11) ist: $\varphi = \varphi_0 + a\,x + b\,y$, wobei $\varphi_0$, a und b Parameter sind, die das affine Verhältnis definieren, wobei die Wahl dieser Parameter die Bestimmung der räumlichen Periode der Modulation der Ultraschallwelle in der bzw. den orthogonal zur Ausbreitungsrichtung (Dz) liegenden Ausrichtungsrichtung(en) ermöglicht.

14. Akusto-optisches Bildgebungsverfahren nach einem der Ansprüche 12 bis 13, **dadurch gekennzeichnet, dass** die Ultraschallwandler (1, 11) ausgerichtet und nur in der ersten Ausrichtungsrichtung (Dx) bzw. der zweiten Ausrichtungsrichtung (Dy) verteilt sind, wobei der Parameter b bzw. a null ist, und dadurch, dass sich die ebene Ultraschallwelle in einer durch die Ausbreitungsrichtung (Dz) und die erste Ausrichtungsrichtung (Dx) bzw. die zweite Ausrichtungsrichtung (Dy) definierten Bildgebungsebene ausbreitet.

15. Akusto-optisches Bildgebungsverfahren nach einem der Ansprüche 12 bis 13, **dadurch gekennzeichnet, dass** die Ultraschallwandler (1, 11) ein Netz in der durch die erste und zweite Ausrichtungsrichtung (Dx, Dy) definierten Ebene bilden und dass sich die ebene Ultraschallwelle in einem durch die Ausbreitungsrichtung (Dz) und die erste und zweite Ausrichtungsrichtung (Dx, Dy) definierten Bildgebungsvolumen ausbreitet.

16. Akusto-optisches Bildgebungsverfahren nach einem der Ansprüche 12 bis 15, **dadurch gekennzeichnet, dass** das zeitlich modulierte Signal ein Rechtecksignal oder ein Sinussignal ist.

17. Akusto-optisches Bildgebungsverfahren nach Anspruch 16, **dadurch gekennzeichnet, dass**:

- das Bildgebungsverfahren die Messung von Fourier-Komponenten des akusto-optischen Bildes des Beobachtungsbereichs ermöglicht, die einer ersten räumlichen Frequenz ($v_x$) in der ersten Ausrichtungsrichtung (Dx), einer zweiten räumlichen Frequenz ($v_y$) in der zweiten Ausrichtungsrichtung (Dy) und einer dritten räumlichen Frequenz ($v_z$) in der Ausbreitungsrichtung (Dz) zugeordnet sind,
- das zeitlich modulierte Signal ein periodisches Rechteckwellensignal ist, dessen zeitliche Frequenz (f) gleich dem Produkt aus der räumlichen Frequenz $v_z$ und der Ausbreitungsgeschwindigkeit (u) der ebenen Ultraschallwelle im Medium ist, wobei sich die Werte +1 und 0 bei jeder halben zeitlichen Periode ($1/(2f)$) abwechseln,
- die Phasenverschiebung ($\varphi$) eine affine Funktion der ersten und zweiten Koordinaten (x, y) der Ultraschallwandler ($\varphi = \varphi_0 - 2n\,v_x\,x - 2n\,v_y\,y$) ist,
- das aus dem Detektionsschritt hervorgegangene akusto-optische Erfassungssignal (9) direkt proportional zur Lichtleistung der markierten Photonen ist, die im Detektionsschritt erkannt wurden,
- der Verarbeitungsschritt darin besteht, das aus dem Detektionsschritt hervorgegangene akusto-optische Erfassungssignal mit einer zeitlichen Demodulationsfunktion zu multiplizieren und das Ergebnis in ein vorbestimmtes Zeitintervall zu integrieren, das ein Vielfaches der zeitlichen Modulationsperiode (1/f) ist, um ein demoduliertes Signal zu erhalten,
- die Fourier-Komponente des akusto-optischen Bildes des Beobachtungsbereichs, die einer ersten, zweiten und dritten räumlichen Frequenz ($v_x$, $v_y$, $v_z$) zugeordnet ist, aus einem demodulierten Signal oder mehreren demodulierten Signalen erhalten wird, die verschiedenen Werten des Parameters $\varphi_0$ entsprechen.

18. Akusto-optisches Bildgebungsverfahren nach Anspruch 17, **dadurch gekennzeichnet, dass** die zeitliche Demodulationsfunktion $\exp(-2n\,j\,f\,t)$ ist, wobei das demodulierte Signal dann eine komplexe Zahl ist, und die Fourier-Komponente einfach proportional zum demodulierten Signal ist, das für $\varphi_0 = n/2$ erhalten wird.

19. Akusto-optisches Bildgebungsverfahren nach Anspruch 17, **dadurch gekennzeichnet, dass** die zeitliche Demodulationsfunktion $\sin(2\pi\,f\,t)$ ist, wobei die Fourier-Komponente dann proportional zur Summe aus dem für $\varphi_0 = 0$ erhaltenen demodulierten Signal und aus j (imaginäre Zahl) multipliziert mit dem für $\varphi_0 = -\pi/2$ erhaltenen demodulierten Signal ist.

20. Akusto-optisches Bildgebungsverfahren nach Anspruch 16, **dadurch gekennzeichnet, dass**:

- die Position jedes Ultraschallwandlers (1, 11) durch eine erste Koordinate (x) in einer ersten orthogonal zur Ausbreitungsrichtung (Dz) liegenden Ausrichtungsrichtung (Dx) und einer zweiten Koordinate (y) in einer zweiten orthogonal zur Ausbreitungsrichtung (Dz) und zur ersten Ausrichtungsrichtung (Dx) liegenden Ausrichtungsrichtung (Dy) definiert wird,
- das Bildgebungsverfahren die Messung von Fourier-Komponenten des akusto-optischen Bildes des Beobachtungsbereichs ermöglicht, die einer ersten räumlichen Frequenz ($v_x$) in der ersten Ausrichtungsrichtung (Dx), einer zweiten räumlichen Frequenz ($v_y$) in der zweiten Ausrichtungsrichtung (Dy) und einer dritten räumlichen Frequenz ($v_z$) in der Ausbreitungsrichtung (Dz) zugeordnet sind,

- das zeitlich modulierte Signal ein periodisches Rechteckwellensignal ist, dessen zeitliche Frequenz (f) gleich der Hälfte des Produkts aus der räumlichen Frequenz $v_z$ und der Ausbreitungsgeschwindigkeit (u) der Ultraschallwelle im Medium ist, wobei sich die Werte -1 und +1 bei jeder halben zeitlichen Periode (1/(2f)) abwechseln,

- die Phasenverschiebung ($\varphi_0$) eine affine Funktion der ersten und zweiten Koordinaten (x, y) der Ultraschallwandler (cp = $\varphi_0$ - $\pi$ $v_x$ x - $\pi$ $v_y$ y) ist,

- das aus dem Detektionsschritt hervorgegangene akusto-optische Erfassungssignal (19) ein interferometrisches Signal zwischen markierten Photonen ist, die im Detektionsschritt erkannt werden, und einer Referenzlichtwelle derselben Wellenlänge wie die in den Beobachtungsbereich einfallende Lichtwelle,

- der Verarbeitungsschritt darin besteht, das aus dem Detektionsschritt hervorgegangene akusto-optische Erfassungssignal mit einer zeitlichen Demodulationsfunktion zu multiplizieren und das Ergebnis in ein vorbestimmtes Zeitintervall zu integrieren, das ein Vielfaches der halben zeitlichen Periode der zeitlichen Modulation (1/2f) ist, um ein demoduliertes Signal zu erhalten,

- das demodulierte Signal mit sich selbst oder mit einem zuvor unter identischen Bedingungen aufgezeichneten demodulierten Signal multipliziert wird, um ein quadriertes demoduliertes Signal zu erhalten, das eine Menge proportional zur Lichtenergie der im Detektionsschritt während des vorbestimmten Zeitintervalls erkannten markierten Photonen ist,

- die Fourier-Komponente des akusto-optischen Bildes des Beobachtungsbereichs, die einer ersten, zweiten und dritten räumlichen Frequenz ($v_x$, $v_y$, $v_z$) zugeordnet ist, durch lineare Kombination der quadrierten demodulierten Signale, die verschiedenen Werten des Parameters $\varphi_0$ entsprechen, erhalten wird.

21. Akusto-optisches Bildgebungsverfahren nach Anspruch 20, **dadurch gekennzeichnet, dass** die zeitliche Demodulationsfunktion $\sin(2\pi f t)$ multipliziert mit $\cos(2\pi f_{us} t + \psi)$ ist, wobei fus die Frequenz der Trägerwelle des Ultraschallfrequenz-Steuersignals und $\psi$ eine Phasenverschiebung ist, wobei die Fourier-Komponente dann proportional zur Summe aus dem für $\varphi_0 = 0$ erhaltenen demodulierten quadrierten Signal und aus j (imaginäre Zahl) multipliziert mit dem für $\varphi_0 = -\pi/4$ erhaltenen demodulierten quadrierten Signal ist, von dem die Summe aus dem für $\varphi_0 = +\pi/2$ erhaltenen quadrierten demodulierten Signal und von j (imaginäre Zahl) multipliziert mit dem für $\varphi_0 = +\pi/4$ erhaltenen quadratierten demodulierten Signal abgezogen wird.

22. Akusto-optisches Bildgebungsverfahren nach Anspruch 21, **dadurch gekennzeichnet, dass** die Bildrekonstruktion durch Fourier-Umkehrtransformation unter Verwendung eines Durchschnitts von Fourier-Komponenten durchgeführt wird, die für verschiedene Werte der y-Phasenverschiebung erhalten wurden, wobei ansonsten alles andere gleich bleibt.

**Claims**

1. System for acousto-optical imaging of an observation zone (3, 13) of a medium, comprising :

- a set of ultrasonic transducers (1, 11) aligned along at least one alignment direction (Dx, Dy), able to produce together an ultrasonic wave (2, 12) in the observation zone (3, 13), propagating along a propagation direction (Dz) orthogonal to the alignment direction(s) (Dx, Dy) of the ultrasonic transducers (1, 11);

- a light source (4, 14) producing an incident light wave (5, 15), meeting said ultrasonic wave in the observation zone to produce tagged photons (6, 16);

- a control device (7, 17) configured to drive each ultrasonic transducer (1, 11) with an ultrasonic frequency control signal (fus);

- a detection system (8, 18) for some of the tagged photons, said tagged photons carrying a time modulation transmitted by the ultrasonic wave encountering the light wave;

- means (10, 110) for processing an acousto-optic acquisition signal (9, 19) received from the detection system, capable of demodulating said acousto-optic acquisition signal;

**characterized in that** the control device (7, 17) is configured to :

- drive each ultrasonic transducer (1, 11) by the control signal, said control signal consisting of an ultrasonic frequency carrier wave (fus),

the carrier wave being identical for all transducers so as to generate an unfocused plane wave propagating in the direction (Dz),

this unfocused plane wave being modulated by a periodic time modulation signal of given temporal frequency and of the same form for all the ultrasonic transducers (1, 11),
this time modulation signal having a phase shift ($\varphi$) whose value is specific to each ultrasonic transducer and is a function of the position of the ultrasonic transducers (1, 11), so that the ultrasonic plane wave emitted by all the ultrasonic transducers (1, 11) has a modulation, which has a spatial periodicity along the alignment direction(s) (Dx, Dy) of the transducers, the value of which is determined by the function between the phase shift ($\varphi$) and the position of the ultrasonic transducers,

- generate different unfocused plane ultrasonic waves propagating in the same direction (Dz) orthogonal to the alignment direction(s) (Dx, Dy) of the ultrasonic transducers (1, 11), and modulated by a periodic time modulation signal of given time frequency and of the same shape for all the ultrasonic transducers (1, 11),

for different values of the given time frequency of the time modulation signal,
with, for each given temporal frequency, different functions relating the phase shift ($\varphi$) and the position of the ultrasonic transducers (1, 11), the different ultrasonic waves corresponding to the different functions relating the phase shift ($\varphi$) and the position of the ultrasonic transducer,
so that the processing means (10, 110) :

- determines Fourier components of an acousto-optical image (21, 121) of the observation zone (3, 13), and
- spatially reconstructs said acousto-optical image (21, 121) by inverse Fourier transformation of the Fourier components.

2. Acousto-optical imaging system according to claim 1, **characterized in that** the position of each ultrasonic transducer (1, 11) is defined by a first coordinate (x) along a first alignment direction (Dx) orthogonal to the propagation direction (Dz) and a second coordinate (y) along a second alignment direction (Dy) orthogonal to the propagation direction (Dz), the phase shift ($\varphi$) is an affine function of the first and second coordinates (x, y) of the ultrasonic transducers (1, 11) of the form $<p = \varphi_0 + a\,x + b\,y$ , where $\varphi_0$, a and b are parameters defining the affine relationship, the choice of these parameters making it possible to determine the spatial period of the modulation of the plane ultrasonic wave along the alignment direction(s) of the transducers (1, 11) orthogonal to the propagation direction (Dz).

3. Acousto-optical imaging system according to claim 2, **characterized in that** the ultrasonic transducers (1, 11) are aligned and distributed solely along the first alignment direction (Dx), the parameter b being zero, and **in that** the plane ultrasonic wave propagates in an imaging plane defined by the propagation direction (Dz) and the first alignment direction (Dx).

4. Acousto-optical imaging system according to one of claims 1 to 2, **characterized in that** the ultrasonic transducers (1, 11) form an array in the plane defined by the first and second alignment directions (Dx, Dy), and **in that** the plane ultrasonic wave propagates in an imaging volume defined by the propagation direction (Dz) and the first and second alignment directions (Dx, Dy).

5. Acousto-optic imaging system according to one of claims 1 to 4, **characterized in that** the time modulation signal is a square-wave or sinusoidal signal.

6. Acousto-optical imaging system according to claim 5, **characterized in that**:

- the position of each ultrasonic transducer is defined by a first coordinate (x) along a first alignment direction (Dx) orthogonal to the propagation direction and a second coordinate (y) along a second alignment direction (Dy) orthogonal to the propagation direction (Dz) and to the first alignment direction (Dx),
this imaging system measures Fourier components of the acousto-optical image of the observation zone associated with a first spatial frequency ($v_x$) along the first alignment direction (Dx), a second spatial frequency ($v_y$) along the second alignment direction (Dy), and a non-zero third spatial frequency ($v_z$) along the propagation direction (Dz),
- the time modulation signal is a periodic square-wave signal, whose temporal frequency (f) is equal to the product of the third spatial frequency ($v_z$) and the propagation velocity (u) of the ultrasonic plane wave in the medium, alternating between +1 and 0 at each half-period (1/(2f)),
- the phase shift ($\varphi$) is an affine function of the first and second ultrasonic transducer coordinates (x, y) of the form $\varphi = \varphi_0 - 2\pi\,v_x\,x - 2\pi\,v_y\,y,$

- the acousto-optic acquisition signal (9) received from the detection system is directly proportional to the luminous power of the marked photons detected by the detection system,
- the processing means multiplies the acousto-optic acquisition signal received from the detection system by a temporal demodulation function (20), integrates the result over a predetermined time interval which is a multiple of the temporal period of the temporal modulation (1/f), to obtain a demodulated signal,
- the Fourier component of the acousto-optical image of the observation zone associated with a first, second and third spatial frequency ($v_x$, $v_y$, $v_z$) is obtained from one or more demodulated signals corresponding to different values of parameter $\varphi_0$.

7.  Acousto-optical imaging system according to claim 6, **characterized in that** the temporal demodulation function is exp(-2rr j f t), the demodulated signal then being a complex number, and the Fourier component being simply proportional to the demodulated signal obtained for $\varphi_0 = \pi/2$ .

8.  Acousto-optic imaging system according to claim 6, **characterized in that** the time demodulation function is sin(2rr f t), the Fourier component then being proportional to the sum of the demodulated signal obtained for $\varphi_0 = 0$ and j (imaginary number) multiplied by the demodulated signal obtained for $\varphi_0 = -(\pi/2)$.

9.  Acousto-optical imaging system according to claim 5, **characterized in that**:

    - the position of the ultrasonic transducer is defined by a first coordinate (x) along a first alignment direction (Dx) orthogonal to the propagation direction (Dz) and a second coordinate (y) along a second alignment direction (Dy) orthogonal to the propagation direction (Dz) and to the first alignment direction (Dx),
    - this imaging system measures Fourier components of the acousto-optical image of the observation zone associated with a first spatial frequency ($v_x$) associated with the first alignment direction (Dx), a second spatial frequency ($v_y$) associated with the second alignment direction (Dy), and a non-zero third spatial frequency ($v_z$) associated with the propagation direction (Dz),
    - the time modulation signal is a periodic square-wave signal, the time frequency (f) of which is equal to half the product of the spatial frequency $v_z$ and the propagation velocity (u) of the ultrasound wave in the medium, alternating between the values -1 and +1 every half time period (1/(2f)),
    - the phase shift ($\varphi$) is an affine function of the first and second ultrasonic transducer coordinates (x, y) of the form $\varphi = \varphi_0 - \pi\, v_x\, x - \pi\, v_y\, y$,
    - the acousto-optical acquisition signal (19) received from the detection system is an interferometric signal between tagged photons detected by the detection system and a reference light wave (122) of the same wavelength as the light wave incident in the observation zone,
    - the processing means multiplies the acousto-optic acquisition signal received from the detection system by a temporal demodulation function (120), integrates the result over a predetermined time interval which is a multiple of the temporal half-period of the temporal modulation (1/(2f)), to obtain a demodulated signal,
    - the demodulated signal is multiplied by itself, or by a demodulated signal previously recorded under identical conditions, to obtain a squared demodulated signal, which is a quantity proportional to the light energy of the marked photons detected by the detection system during the predetermined time interval,
    - the Fourier component of the acousto-optical image of the observation zone associated with a first, second and third spatial frequency ($v_x$, $v_y$, $v_z$) is obtained from a plurality of demodulated squared signals corresponding to different values of parameter $\varphi_0$.

10. Acousto-optic imaging system according to claim 9, **characterized in that** the temporal demodulation function is sin(2rr f t) multiplied by $\cos(2\pi\, fus\, t + \varphi)$, where fus is the frequency of the carrier wave of the ultrasonic frequency control signal and $\varphi$ a phase, said Fourier component then being proportional to the sum of the demodulated squared signal obtained for $\varphi_0 = 0$ and j (imaginary number) multiplied by the demodulated squared signal obtained for $\varphi_0 = -(\pi/4)$, minus the sum of the demodulated squared signal obtained for $\varphi_0 = +(\pi/2)$ and j (imaginary number) multiplied by the demodulated squared signal obtained for $\varphi_0 = -+(\pi/4)$.

11. Acousto-optic imaging system according to claim 10, **characterized in that** inverse Fourier transform image reconstruction is performed using an average of Fourier components obtained for different $\Psi$ phase values, all other things being equal.

12. A method of acousto-optical imaging of an observation zone (3, 13) of a medium, the method comprising:

    - E1: a step of generating a light wave (5, 15) incident in the observation zone,

- E2: a step of generating, by means of a set of ultrasonic transducers (1, 11) aligned along at least one alignment direction, an ultrasonic wave (2, 12) in the observation zone, with an ultrasonic frequency (fus), propagating along a propagation direction (Dz) orthogonal to the at least one alignment direction(s) of the ultrasonic transducers (1, 11),

- E3: a step for generating tagged photons (6, 16) carrying a time modulation transmitted by the ultrasonic wave encountering the light wave,

- E4: a step of detecting some of the marked photons and generating an acousto-optic acquisition signal (9, 19) by a detection system (8, 18),

- E5: a step of processing said acousto-optical acquisition signal provided by said detection system comprising a step of temporally demodulating said acousto-optical acquisition signal using a temporal demodulation signal (20, 120),

**characterized in that**:

(i) in the generation step (E2), all ultrasonic transducers (1, 11) are driven by a control signal consisting of an ultrasonic frequency carrier wave (fus),

the carrier wave being identical for all ultrasonic transducers (1, 11), so as to generate an unfocused plane wave propagating in the direction (Dz) orthogonal to the alignment direction(s) (Dx, Dy) of the ultrasonic transducers (1, 11),

this unfocused plane wave being modulated by a periodic time modulation signal of given time frequency and of the same form for all the ultrasonic transducers (1, 11),

this time modulation signal having a phase shift ($\varphi$) whose value is specific to each ultrasonic transducer and which is a function of the position of the ultrasonic transducers (1, 11),

so that the ultrasonic plane wave emitted by all the ultrasonic transducers has a modulation, which has a spatial periodicity along the direction(s) of alignment of the transducers, the value of the spatial period of which is determined by the function between the phase shift ($\varphi$) and the position of the ultrasonic transducers (1, 11);

(ii) the method further comprising a step E6 in which the preceding steps E1 to E5 are repeated several times, for different values of the given temporal frequency of the time modulation signal,

with, for each given temporal frequency, different functions relating the phase shift ($\varphi$) and the position of the ultrasonic transducers (1, 11), the different unfocused ultrasonic waves propagating in the same direction (Dz) orthogonal to the alignment direction(s) (Dx, Dy) of the ultrasonic transducers (1, 11), corresponding to the different functions relating the phase shift ($\varphi$) and the position of the ultrasonic transducer,

so as to determine Fourier components of an acousto-optical image (21, 121) of the observation zone (3, 13), and to spatially reconstruct said acousto-optical image (21, 121) by inverse Fourier transformation of the Fourier components.

13. Acousto-optical imaging method according to claim 12, **characterized in that** the position of each ultrasonic transducer (1, 11) is defined by a first coordinate (x) along a first alignment direction (Dx) orthogonal to the propagation direction (Dz) and a second coordinate (y) along a second alignment direction (Dy) orthogonal to the propagation direction (Dz) and the first alignment direction (Dx), the phase shift ($\varphi$) is an affine function of the first and second coordinates (x, y) of the ultrasonic transducers (1, 11): $\varphi = \varphi_0 + a\,x + b\,y$, where $\varphi_0$, a and b are parameters defining the affine relationship, the choice of these parameters making it possible to determine the spatial period of the ultrasonic wave modulation along the alignment direction(s) orthogonal to the propagation direction (Dz).

14. Acousto-optical imaging method according to one of claims 12 to 13, **characterized in that** the ultrasonic transducers (1, 11) are aligned and uniquely distributed along the first alignment direction (Dx), respectively the second alignment direction (Dy), the parameter b, respectively a, being zero, and **in that** the ultrasonic plane wave propagates in an imaging plane defined by the propagation direction (Dz) and the first alignment direction (Dx), respectively the second alignment direction (Dy).

15. Acousto-optical imaging method according to one of claims 12 to 13, **characterized in that** the ultrasonic transducers (1, 11) form an array in the plane defined by the first and second alignment directions (Dx, Dy) and **in that** the plane ultrasonic wave propagates in an imaging volume defined by the propagation direction (Dz) and the first and second alignment directions (Dx, Dy).

16. Acousto-optical imaging method according to one of claims 12 to 15, **characterized in that** the time modulation signal is a square-wave signal or a sinusoidal signal.

17. Acousto-optical imaging method according to claim 16, **characterized in that**:

    - the imaging method enables measurement of Fourier components of the acousto-optical image of the observation zone associated with a first spatial frequency ($v_x$) along the first alignment direction (Dx), a second spatial frequency ($v_y$) along the second alignment direction (Dy), and a non-zero third spatial frequency ($v_z$) along the propagation direction (Dz),

    - the time modulation signal is a periodic square-wave signal, whose temporal frequency (f) is equal to the product of the spatial frequency $_{nz}$ and the propagation velocity (u) of the ultrasonic plane wave in the medium, alternating between +1 and 0 at each temporal half-period (1/(2f)),

    - the phase shift ($\varphi$) is an affine function of the first and second coordinates (x, y) of the ultrasonic transducers ($\varphi = \varphi_0 - 2\pi v_x x - 2\pi v_y y$),

    - the acousto-optic acquisition signal (9) resulting from the detection step is directly proportional to the luminous power of the marked photons detected in the detection step,

    - the processing step consists in multiplying the acousto-optical acquisition signal from the detection step by a temporal demodulation function, and integrating the result over a predetermined time interval which is a multiple of the temporal modulation period (1/f), to obtain a demodulated signal,

    - the Fourier component of the acousto-optical image of the observation zone associated with a first, second and third spatial frequency ($v_x$, $v_y$, $v_z$) is obtained from one or more demodulated signals corresponding to different values of the parameter $\varphi_0$.

18. Acousto-optical imaging method as claimed in claim 17, **characterized in that** the temporal demodulation function is $\exp(-2\pi j f t)$, the demodulated signal being a complex number, and the Fourier component being simply proportional to the demodulated signal obtained for $\varphi_0 = + (\pi/2)$.

19. Acousto-optical imaging method according to claim 17, **characterized in that** the time demodulation function is $\sin(2\pi f t)$, the Fourier component then being proportional to the sum of the demodulated signal obtained for $\varphi_0 = 0$ and j (imaginary number) multiplied by the demodulated signal obtained for $\varphi_0 = - (\pi/2)$.

20. Acousto-optical imaging method according to claim 16, **characterized in that**:

    - the position of each ultrasonic transducer (1, 11) is defined by a first coordinate (x) along a first alignment direction (Dx) orthogonal to the propagation direction (Dz) and a second coordinate (y) along a second alignment direction (Dy) orthogonal to the propagation direction (Dz) and to the first alignment direction (Dx),

    - the imaging process measures Fourier components of the acousto-optical image of the observation zone associated with a first spatial frequency ($v_x$) along the first alignment direction (Dx), a second spatial frequency ($v_y$) along the second alignment direction (Dy), and a non-zero third spatial frequency ($v_z$) along the propagation direction (Dz),

    - the time modulation signal is a periodic square-wave signal, the time frequency (f) of which is equal to half the product of the spatial frequency $v_z$ and the propagation velocity (u) of the ultrasound wave in the medium, alternating between -1 and +1 at each half-time period (1/(2f)),

    - the phase shift ($\varphi$) is an affine function of the first and second coordinates (x, y) of the ultrasonic transducers ($\varphi = \varphi_0 - \pi v_x x - \pi v_y y$),

    - the acousto-optical acquisition signal (19) resulting from the detection step is an interferometric signal between marked photons detected in the detection step and a reference light wave of the same wavelength as the light wave incident in the observation zone,

    - the processing step consists in multiplying the acousto-optical acquisition signal from the detection step by a temporal demodulation function, and integrating the result over a predetermined time interval which is a multiple of the temporal modulation half-period (1 /(2f)), to obtain a demodulated signal,

    - the demodulated signal is multiplied by itself, or by a demodulated signal previously recorded under identical conditions, to obtain a squared demodulated signal, which is a quantity proportional to the light energy of the marked photons detected at the detection stage during the predetermined time interval,

    - the Fourier component of the acousto-optical image of the observation zone associated with a first, second and third spatial frequency ($v_x$, $v_y$, $v_z$) is obtained by linear combination of the squared demodulated signals corresponding to different values of parameter $\varphi_0$.

21. Acousto-optical imaging method according to claim 20, **characterized in that** the temporal demodulation function is $\sin(2p\,f\,t)$ multiplied by $\cos(2p\,f_{US}\,t+\Psi)$, where fus is the frequency of the carrier wave of the ultrasonic frequency control signal and $\Psi$ a phase shift, the Fourier component then being proportional to the sum of the demodulated squared signal obtained for $\varphi_0=0$ and j (imaginary number) multiplied by the demodulated squared signal obtained for $\varphi_0=-(\pi/4)$, minus the sum of the demodulated squared signal obtained for $\varphi_0=+(\pi/2)$ and j (imaginary number) multiplied by the demodulated squared signal obtained for $\varphi_0=+(\pi/4)$.

22. Acousto-optic imaging method as claimed in claim 21, **characterized in that** the inverse Fourier transform image reconstruction is performed using an average of Fourier components obtained for different values of the phase shift $\Psi$, all other things being equal.

EP 3 820 354 B1

**FIG. 1**

FIG. 2

FIG. 3

## FIG. 4a

## FIG. 4b

# FIG. 5

FIG. 6a

FIG. 6b

EP 3 820 354 B1

**EP 3 820 354 B1**

**RÉFÉRENCES CITÉES DANS LA DESCRIPTION**

**Documents brevets cités dans la description**

- WO 2016193554 A1
- FR 1052535
- FR 2958430 A1